# EUROPEAN PATENT APPLICATION

(11) **EP 3 009 422 A1**
(43) Date of publication of application: **20.04.2016**
(21) Application number: 14188799.2
(22) Date of filing: 14.10.2014
(51) Int. Cl.: C07C 311/58, C07C 311/59, C07D 285/135, A61K 31/64, A61P 3/10

(54) **Photo-switchable sulfonylureas and their uses**

(71) Applicant: Ludwig-Maximilians-Universität München, 80539 München (DE)
(72) Inventor: The designation of the inventor has not yet been filed
(74) Representative: Engelhard, Markus

(57) **Abstract**

The present invention relates to sulfonylurea analogous compounds comprising a sulfonylurea moiety and a photoresponsive moiety. The present invention further relates to methods for generating the sulfonylurea analogous compounds of the invention and to pharmaceutical compositions comprising at least one sulfonylurea analogous compound of the invention. The present invention relates to the sulfonylurea analogous compounds or the pharmaceutical composition of the invention for use in medicine, in particular for use as anti-diabetic agent and/or in the prevention and/or treatment of diabetes conditions or a disease state requiring modulation of ATP-sensitive potassium (K⁺) (K_{ATP}) channels. The present invention also relates to the use of the sulfonylurea analogous compounds for regulating ATP-sensitive potassium (K⁺) (K_{ATP}) channels and/or for assessing the functional role and/or activation of K_{ATP} channels.

## Description

The present invention relates to sulfonylurea analogous compounds comprising a sulfonylurea moiety and a photoresponsive moiety. The present invention further relates to methods for generating the sulfonylurea analogous compounds of the invention and to pharmaceutical compositions comprising at least one sulfonylurea analogous compound of the invention. The present invention relates to the sulfonylurea analogous compounds or the pharmaceutical composition of the invention for use in medicine, in particular for use as anti-diabetic agent and/or in the prevention and/or treatment of diabetes conditions or a disease state requiring modulation of ATP-sensitive potassium (K⁺) (K_{ATP}) channels. The present invention also relates to the use of the sulfonylurea analogous compounds for regulating ATP-sensitive potassium (K⁺) (K_{ATP}) channels and/or for assessing the functional role and/or activation of K_{ATP} channels.

### BACKGROUND OF THE INVENTION

Type 2 diabetes mellitus (T2DM) is a global healthcare epidemic associated with life-changing sequelae ranging from blindness to cancer (Currie et al., 2009; Stitt, 2010). This endocrine disease, which currently affects 1 in 12 of the adult population worldwide, involves a disturbance of normal glucose homeostasis due to failure of the pancreatic beta cell mass to adequately compensate for increased peripheral insulin resistance (Prentki & Nolan, 2006). As such, the rescue of insulin release through the coaxing of beta cell activity remains a therapeutically desirable approach for the long-term restoration of normal glucose levels.

Sulfonylureas, which target ATP-sensitive potassium (K⁺) (K_{ATP}) channels, are a mainstay of diabetes therapy (see e.g. Fineman et al., 2003). K_{ATP} channels are hetero-octameric structures composed of four regulatory sulfonylurea receptor subunits (SUR1) and four Kir6.2 subunits, the latter forming a central ion pore that permits K⁺ efflux_ENREF_7 (see e.g. Miki et al., 1999). By binding to SUR1, sulfonylureas block the Kir6.2 inward rectifier, leading to cell depolarization and opening of voltage-dependent Ca²⁺ channels (VDCC) (see e.g. Seino & Miki, 2003). The ensuing Ca²⁺ influx (Rorsman et al., 2012) along with K_{ATP} channel-independent signals_ENREF_14 (Henquin, 2009), drives various downstream processes which ultimately converge on the exocytosis of insulin. Elevated circulating insulin can then act on target tissues to improve glucose uptake, hepatic glycogenesis and fatty acid synthesis.

While sulfonylureas are widely prescribed because of their effectiveness and relative inexpensiveness, they have a range of off-target effects which limits their therapeutic use. For example, sulfonylureas can provoke prolonged episodes of low blood glucose due to hyperinsulinemia_ENREF_17 (Jennings et al., 1989), elevate cardiovascular disease risk (Evans et al., 2006), and induce weight gain (Nathan et al., 2006). Conversely, there is a lack of tools for the precise functional dissection of K_{ATP} channels located not only in the pancreas, but also in the brain (Lam et al., 2010; Hernandez-Sanchez et al., 2001), heart_ENREF_22 (Engler & Yellon, 1996) and vascular smooth muscle (Quayle et al., 1997).

There is a need in the art for improved sulfonylurea compounds.

### SUMMARY OF THE INVENTION

According to the present invention this object is solved by a sulfonylurea analogous compound comprising a sulfonylurea moiety and a photoresponsive moiety, preferably having the general formula I

**Ph - SU** (I)

wherein
**Ph** is the photoresponsive moiety, and
**SU** is the sulfonylurea moiety.

According to the present invention this object is solved by a method for synthesizing/generating the sulfonylurea analogous compounds of the present invention, comprising at least one of the following
the use of sulfanilamide and *N,N*-diethylaniline as the starting compounds,
the use of sulfanilamide and phenol as the starting compounds,
the use of 5-amino-1,3,4-thiadiazole-2-sulfonamide and *N,N*-diethylaniline as the starting compounds,
the use of nitroso compounds with anilines,
the use of hydrazines and coupling partners using Pd-chemistry,
via sulfonamide azobenzenes,
the further derivatization to a sulfonylurea by the use of aliphatic isocyanates (e.g. cyclohexyl, *n*-butyl, *trans*-4-methyl-cyclohexyl)
and/or
the use of any sulfonylchlorides thereof together with ureas.

According to the present invention this object is solved by a pharmaceutical composition, comprising
(i) at least one sulfonylurea analogous compound according to the present invention,
(ii) optionally, pharmaceutically acceptable excipient(s) and/or carrier(s),
(iii) optionally, further compound(s).

According to the present invention this object is solved by the sulfonylurea analogous compounds of the present invention or the pharmaceutical compositions of the present invention for use in medicine.

According to the present invention this object is solved by the sulfonylurea analogous compounds of the present invention for use as anti-diabetic agent.

According to the present invention this object is solved by the sulfonylurea analogous compounds of the present invention or the pharmaceutical compositions of the present invention for use in the prevention and/or treatment of diabetes conditions or a disease state requiring modulation of ATP-sensitive potassium (K⁺) (K_{ATP}) channels.

According to the present invention this object is solved by using the sulfonylurea analogous compounds of the present invention for regulating ATP-sensitive potassium (K⁺) (K_{ATP}) channels and/or for assessing the functional role and/or activation of ATP-sensitive potassium (K⁺) (K_{ATP}) channels.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

### Photo-switchable sulfonylurea compounds

As discussed above, the present invention provides sulfonylurea analogous compounds comprising a sulfonylurea moiety and a photoresponsive moiety.

The sulfonylurea analogous compound of the present invention preferably has the general formula I

**Ph - SU** (I)

wherein
**Ph** is the photoresponsive moiety, and
**SU** is the sulfonylurea moiety.

The term "sulfonylurea analogous compound" or "photo-switchable sulfonylurea compound" when used herein refers to a compound that has the same or similar biologic activity or action mechanism as sulfonylureas known in the art as anti-diabetic drugs (i.e. the sulfonylurea analogous compound/ photo-switchable sulfonylurea compound, as well as sulfonylureas bind to an ATP-dependent K⁺(K_{ATP}) channel on the cell membrane of pancreatic beta and alpha cells and thus act by increasing insulin release and decreasing glucagon release). The sulfonylurea analogous compounds or photo-switchable sulfonylurea compounds of the present invention comprise a further moiety which allows the compound to be activated or inactivated by irradiation with light, or by illumination and dark relaxation.

The term "photoresponsive moiety" when used herein refers a moiety, preferably an azobenzene or a derivative thereof, that is a chemical unit that is responsive to light. The wavelength of the light response is determined by the electronic structure of the photoresponsive moiety, preferably the azobenzene or a derivative thereof, which can easily be modified/tuned.

The term "sulfonylurea moiety" when used herein refers to the pharmacological unit having the structure R-SO₂-NH-C(=O)-NH-R' or R-SO₂-NH-C(=O)-NH-R₂, which is common to the sulfonylurea drug family.
For example, the following (wherein R comprises an aromatic moiety):

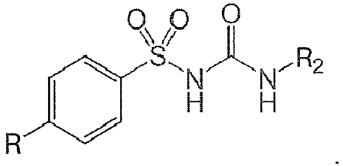

Preferably, the photoresponsive moiety undergoes *trans-cis* isomerization by excitation with light, or by illumination and dark relaxation.
In particular, the photoresponsive moiety undergoes *cis*-isomerization by excitation with light and *trans*-isomerization by illumination and/or dark relaxation.
This intrinsic property can be used to induce a conformational change in the molecule and change binding affinity/efficacy towards a biological target accordingly.

In a preferred embodiment, the photoresponsive moiety is an azobenzene or a derivative thereof.
Examples for azobenzene derivatives are:
- heterocyclic azobenzene structure(s),
- substituted azobenzene structure(s) (e.g. tetra-ortho),
- bridged azobenzenes (e.g. by an o,o'-ethylene chain).

Preferably,
- the *cis* state or isomer is active / is in the binding conformation to close ATP-sensitive potassium (K⁺) (K_{ATP}) channels,
   and
- the *trans* state or isomer is inactive.

In an embodiment, the reverse is also applicable,
- the *cis* state or isomer is inactive ,
   and
- the *trans* state or isomer is active / is in the binding conformation to close ATP-sensitive potassium (K⁺) (K_{ATP}) channels,.

K_{ATP} channels are ubiquitously-expressed ion channels and can be found in the pancreas, brain, muscle and heart, where they generally serve to link metabolism to cell activity (Engler & Yellon, 1996; Quayle et al.; 1997; Miki et al., 1999; Hernandez-Sanchez et al., 2001; Pocai et al., 2005; Lam et al., 2010). Their function is extensively detailed in pancreatic beta cells. Under resting conditions, K_{ATP} channels permit K⁺ efflux, hypopolarizing the cell membrane and suppressing action potential firing and cell activity. Following a rise in blood glucose concentrations, such as that seen after a meal, the sugar is metabolized, leading to an increase in the cytosolic free ATP:ADP ratio, closure of K_{ATP} channels and membrane depolarization. Voltage-dependent Ca²⁺-channels (VDCC) then open, and the ensuing Ca²⁺-influx drives Ca²⁺-dependent exocytosis of insulin-containing granules *via* interactions with the exocytotic machinery. Insulin then binds its cognate receptor to increase glucose uptake and utilization (see e.g. Rorsman et al., 2012). As well as reduced insulin sensitivity, a hallmark of type 2 diabetes mellitus (T2DM) is defective beta cell function, resulting in impaired insulin secretion. K_{ATP} channel activity has therefore remained a popular target for the restoration of insulin secretion, by allowing defects in metabolism, exocytosis and cell number to be bypassed.

K_{ATP} channels are hetero-octameric structures assembled from four inwardly-rectifying subunits (*e.g.* Kir6.1 and Kir6.2 and four sulfonylurea receptor (SUR) subunits (*e.g.* SUR1 and SUR2A/B in the heart) (see Table below for subunit distribution and function) (see e.g. Miki et al., 1999). Kir6.x possesses two transmembrane regions and comprises the ion pore for K⁺ efflux. In the pancreatic beta cell, Kir6.2 primarily transduces alterations in ATP:ADP to channel state *via* interactions between the β- and γ-phosphates of the nucleotide and N- and C- termini of the subunit. By contrast, SURs possess three transmembrane regions and, in the beta cell, SUR1 contains nucleotide binding pockets on the cytoplasmic portion which modulate ATP-sensitivity of the K_{ATP} channel complex. Importantly, the SUR also confers sensitivity to sulfonylurea drugs and sulfonylurea binding rapidly induces channel closure (Inagaki et al., 1996; Ashfield et al., 1999).

| **Subunits** | **Distribution** | **Role** |
|---|---|---|
| Kir6.2/SUR1 | • Pancreatic beta cell | • Regulation of insulin secretion |
| | • Hypothalamus | • Central glucose homeostasis |
| | • Substantia nigra | • Neuronal firing and behavior |
| Kir6.2/SUR2A | • Cardiac muscle | • Cardiac stress (contractility) |
| | • Skeletal muscle | • Exercise capacity |
| Kir6.2/SUR2B | • Smooth muscle | • Tissue perfusion |
| Kir6.1/SUR2B | • Vasculature | • Sepsis |

In a preferred embodiment, the sulfonylurea moiety is derived from second and/or third generation sulfonylureas.

In a preferred embodiment, the sulfonylurea moiety is derived from glibenclamide and/or glimepiride, and/or the sulfonylurea moiety contains substitutent(s) on the terminal sulfonylurea nitrogen.
Such substitutent(s) can be aliphatic chains and/or rings, which can contain heteroatoms.

Preferably, the sulfonylurea moiety and the photoresponsive moiety are covalently attached to each other.

In one embodiment, the aromatic part of a known sulfonylurea is converted to an azobenzene. In order to be similar to or resemble the structure of the known sulfonylurea glimepiride, furthermore a cyclohexyl group was attached to the azobenzene.

In a preferred embodiment, the sulfonylurea analogous compound of the present invention has the general formula II 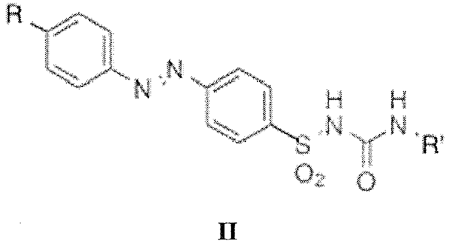 wherein
R is selected from hydrogen, alkyl, alkylamino, hydroxy or substituted hydroxy groups, morpholines and/or piperazines,
   and
R' is selected from cyclohexyl and substituted versions thereof, aliphatic linear chains (such as alkyl) and/or structures containing heteroatoms,

In a preferred embodiment, the sulfonylurea analogous compound of the present invention is (*E*)-*N*-(cyclohexylcarbamoyl)-4-((4-(diethylamino)phenyl)diazenyl)benzenesulfonamide (JB253), which is a sulfonylurea analogous compound of the present invention having general formula II, wherein R is *N,N*-diethylamino- and wherein R' is cyclohexyl.

In a preferred embodiment, the sulfonylurea analogous compound of the present invention is selected from

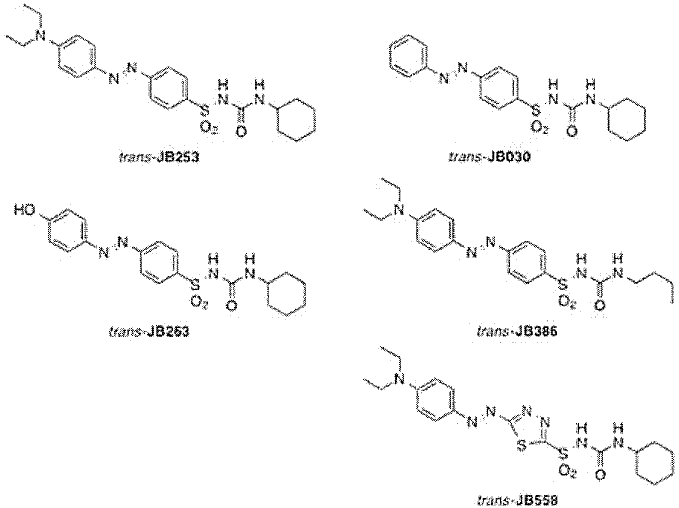

Preferably, the activation wavelength is in the ultraviolet, visible or infrared ranges of the electromagnetic spectrum.

For example, for JB253, the activation wavelength is in the visible light range, such as in the range from about 400 to about 500 nm, while JB030 is activated with UV light (about 350 nm) and JB558 with yellow-orange light (about 530 nm).

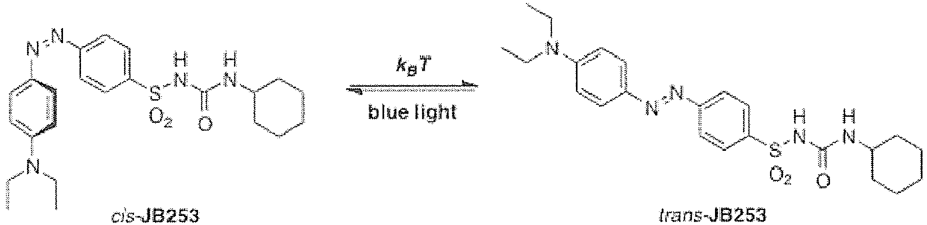

Preferably, the sulfonylurea analogous compound is active under irradiation with light, and inactive under either illumination or in the dark.

A sulfonylurea analogous compound of the invention has preferably at least one of the following characteristics:
(1) it is active under irradiation with light, and inactive under either illumination or in the dark,
(2) rapid conversion into active *cis*-state /conformation,
(3) rapid dark relaxation into *trans*-state,
(4) has a similar EC₅₀ value to the sulfonylurea it is similar to/ its analogue it is (in case of JB253: similar EC₅₀ value to glimepiride)
(5) allows regulation of ATP-sensitive potassium (K⁺) (K_{ATP}) channel activity preferably repeatedly and reversibly (such as by turning on and off of a light source)
(6) it is not cytotoxic
(7) it allows spatially and temporally restricted activation by illumination
(8) can be modified / tailored
   such as regarding
   the activation wave length, and/or
   pharmacological properties (i.e. substitution on the sulfonylurea).

Preferred or advantageous characteristics of the compound JB253 are:
(1) JB253 can be applied/administered exogenously and then washed out.
(2) JB253 allows specific photo switching of K_{ATP} channels.
(3) JB253 is very light sensitive and can be activated with e.g. a LED flashlight (which is about 100 times less intensity than required to activate optogenes).

In an embodiment, the sulfonylurea analogous compound of the present invention can comprise further moiety/moieties or component(s),
such as
- label (e.g. radioisotopes, MRI responsive groups, IR dyes, fluorophores)
- tag(s) (e.g. antibodies, peptides),
- anchoring group(s),
- other pharmacological units (e.g. biguanides, incretins)
and/or
- cell penetrating peptides (e.g. TAT).

As discussed above, the present invention provides pharmaceutical compositions, comprising
(i) at least one sulfonylurea analogous compound according to the present invention,
(ii) optionally, pharmaceutically acceptable excipient(s) and/or carrier(s).

In an embodiment, the pharmaceutical composition of the present invention can comprise further compound(s) or drug(s).

Said further compound(s) or drug(s) can be another anti-diabetic drug(s).

Said further compound(s) or drug(s) can be/include:
- allosteric glucagon-like peptide 1 receptor (GLP-1R) modulators,
- GLP1-R agonists,
- dipeptidyl peptidase-4 (DPP4) inhibitors,
- biguanides,
- peroxisome proliferator-activated receptor gamma (PPAR-γ) agonists,
- sodium-glucose co-transporter 2 (SGLT2) inhibitors,
- oxyntomodullin, and/or
- GLP-1/glucagon co-infusions.

### Method of generating the photo-switchable sulfonylurea compounds

As discussed above, the present invention provides methods for synthesizing/generating the sulfonylurea analogous compounds of the present invention.

Said method comprises at least one of the following
- the use of sulfanilamide and *N,N*-diethylaniline as the starting compounds,
- the use of sulfanilamide and phenol as the starting compounds,
- the use of 5-amino-1,3,4-thiadiazole-2-sulfonamide and *N,N*-diethylaniline as the starting compounds,
- the use of nitroso compounds with anilines,
- the use of hydrazines and coupling partners using Pd-chemistry,
- via sulfonamide azobenzenes,
- the further derivatization to a sulfonylurea by the use of aliphatic isocyanates (e.g. cyclohexyl, *n*-butyl, *trans*-4-methyl-cyclohexyl)
   and/or
- the use of any sulfonylchlorides thereof together with ureas.

In an embodiment, the method is a three-step synthesis:
Commencing/starting with sulfanilamide that is diazotized and the resulting diazonium salt trapped with *N,N*-diethylaniline yielding a sulfonamide, which is further derivatized by using cyclohexyl isocyanate.

### Medical uses of the photo-switchable sulfonylurea compounds

As discussed above, the present invention provides the sulfonylurea analogous compounds of the present invention or the pharmaceutical compositions of the present invention for use in medicine.

As discussed above, the present invention provides the sulfonylurea analogous compounds of the present invention for use as anti-diabetic agent.

As discussed above, the present invention provides the sulfonylurea analogous compounds of the present invention or the pharmaceutical compositions of the present invention for use in the prevention and/or treatment of diabetes conditions or a disease state requiring modulation of ATP-sensitive potassium (K⁺) (K_{ATP}) channels.

Preferably, diabetes or "diabetes conditions" is/refers to/comprises
- type 1 diabetes,
- type 2 diabetes,
- maturity onset diabetes of the young (MODY),
- permanent neonatal diabetes mellitus (PNDM),
- latent onset autoimmune diabetes of adults (LADA),
- any disease state requiring restoration of normal glucose homeostasis,
- any disease state characterized by defective glucose tolerance, glucose counterregulation and/or insulin release.

A "disease state requiring modulation of ATP-sensitive potassium (K⁺) (K_{ATP}) channels" refers to diseases that are either based on intrinsic malfunctioning K_{ATP} channels (e.g. due to mutations) and/or to disease states where signaling to or from K_{ATP} channels is disturbed.

In one embodiment, the use for the prevention and/or treatment according to the invention comprises at least one of
(1) targeting of activity,
(2) spatially and temporally restricted activation by illumination,
(3) optical control of electrical activity,
(4) optical control over calcium fluxes,
(5) optical control over insulin release/secretion,
(6) optical control over glucagon release/secretion,
(7) optical control over somatostatin release/secretion,
(8) optical control over pancreatic peptide release/secretion,
(9) optical control over ghrelin release/secretion,
(10) repeated modulation of any cell/tissue expressing K_{ATP} channels,
(11) pulsatile or oscillatory insulin release/secretion,
(12) pulsatile or oscillatory activation of any cell/tissue expressing K_{ATP} channels,
(13) tailoring of insulin release/secretion,
(14) optical control of exchange protein activated by cAMP (Epac).

In one embodiment, the use for the prevention and/or treatment of diabetes or diabetes conditions comprises at least one of
(1) targeting of activity,
(2) spatially and temporally restricted activation by illumination,
(3) optical control of electrical activity,
(4) optical control over calcium fluxes,
(5) optical control over insulin release/secretion,
(6) optical control over glucagon release/secretion,
(7) optical control over somatostatin release/secretion,
(8) optical control over pancreatic peptide release/secretion,
(9) optical control over ghrelin release/secretion,
(11) pulsatile or oscillatory insulin release/secretion,
(13) tailoring of insulin release/secretion,
(14) optical control of exchange protein activated by cAMP (Epac).

In one embodiment, the use for the prevention and/or treatment of a disease state requiring modulating of ATP-sensitive potassium (K⁺) (K_{ATP}) channels comprises at least one of
(1) targeting of activity,
(2) spatially and temporally restricted activation by illumination,
(10) repeated modulation of any cell/tissue expressing K_{ATP} channels,
(12) pulsatile or oscillatory activation of any cell/tissue expressing K_{ATP} channels.

In one embodiment, the use for the prevention and/or treatment according to the invention comprises photodynamic therapy or photopharmacology.

"Photodynamic therapy" (PDT) as used herein refers to a form of phototherapy using nontoxic light-sensitive compounds (i.e. the sulfonylurea analogous compounds of the present invention) that are exposed selectively to light, whereupon they become active.

PDT is used clinically to treat a wide range of medical conditions, including wet age-related macular degeneration and malignant cancers and has proven ability to kill microbial cells, including bacteria, fungi and viruses, and is recognized as a treatment strategy which is both minimally invasive and minimally toxic.

"Photoparmacology" as used herein refers to the use of light to finely regulate drug activity and modify biological processes accordingly.

Photopharmacology is used to reversibly target and regulate drug activity with the aim of more precisely controlling cell/tissue function, reducing drug side effects/off target activity, and preventing residual drug activity in the environment.

### Further uses

As discussed above, the present invention provides the use of the sulfonylurea analogous compounds of the present invention for regulating ATP-sensitive potassium (K⁺) (K_{ATP}) channels and/or for assessing the functional role and/or activation of ATP-sensitive potassium (K⁺) (K_{ATP}) channels.

### Methods of treatment

The present invention provides a method for the prevention and/or treatment of diabetes conditions or a disease state requiring modulation of ATP-sensitive potassium (K⁺) (K_{ATP}) channels.

Said method comprises the administration of a therapeutically effective amount of at least one sulfonylurea analogous compound of the present invention to a patient or subject in need thereof.

Preferably, diabetes or "diabetes conditions" is/refers to/comprises
- type 1 diabetes,
- type 2 diabetes,
- maturity onset diabetes of the young (MODY),
- permanent neonatal diabetes mellitus (PNDM),
- latent onset autoimmune diabetes of adults (LADA),
- any disease state requiring restoration of normal glucose homeostasis,
- any disease state characterized by defective glucose tolerance, glucose counterregulation and/or insulin release.

### Further description of preferred embodiments

Sulfonylureas are widely prescribed for the treatment of type 2 diabetes mellitus (T2DM). Through their actions on ATP-sensitive potassium (K_{ATP}) channels, sulfonylureas boost insulin release from the pancreatic beta cell mass to restore glucose homeostasis. A limitation of these compounds is the elevated risk of developing hypoglycemia and cardiovascular disease, both potentially fatal complications.

The inventors combined the glucose-lowering attributes of sulfonylureas with the exquisite spatiotemporal control conferred by possession of photoresponsive elements (Fortin et al., 2008; Fehrentz et al., 2011).

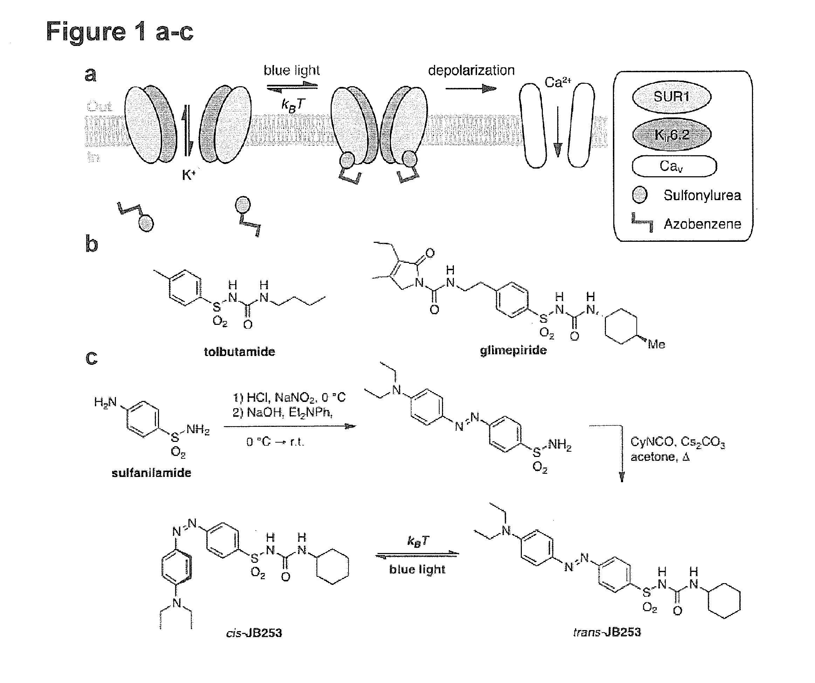

In this patent application, we describe the design and development of photoswitchable sulfonylureas, such as the compound JB253 - a "fourth-generation" sulfonylurea based on glimepiride that bears an azobenzene photoswitch, endowing K_{ATP} channels with remarkable photocontrollable properties (see Figure 1a).

We demonstrate herein that the photoswitchable sulfonylureas according to the invention (such as compound JB253) offer sensitive, reversible and repeated manipulation of K_{ATP} channel state and beta cell activity with visible light. Using *in situ* imaging and hormone assays, we further show that photoswitchable sulfonylureas, in particular compound JB253, bestow light-sensitivity upon rodent and human pancreatic beta cell function. Thus, the photoswitchable sulfonylureas of the present invention, such as compound JB253, enable the optical control of insulin release and offer a valuable research tool for the interrogation of K_{ATP} channel function in health and T2DM. The photoswitchable sulfonylureas according to the invention, such as compound JB253, allow the selective targeting of K_{ATP} channels in the pancreas and elsewhere.

### Discussion

In the present application, we describe the development and testing of photoswitchable sulfonylureas, such as the compound JB253, a chemical chimera of glimepiride and an azobenzene, which allows light-induced closure of K_{ATP} channels. In the primary tissue employed here, *viz* islets of Langerhans, this translates to activated Ca²⁺ flux and insulin release.

The principles of photopharmacology, *i.e.* the control of biological function with small molecule photoswitches, are now well-established (Fehrentz et al., 2011; Gorostiza & Isacoff, 2008; Velema et al, 2014. In particular, azobenzene photoswitches have been employed as photochromic neurotransmitters and neuromodulators (Stein et al., 2012; Tochitsky et al., 2012), ion channel blockers (Fortin et al., 2008; Mourot et al., 2012), covalently-bound ion channel gates (Lemoine et al., 2013) and enzyme inhibitors (Velema et al., 2013; Broichhagen et al., 2014). With respect to ion channels, however, they have mostly been used to optically control excitable cells in the mammalian nervous system, and none have directly targeted K_{ATP} channels. These are ubiquitously-expressed channels that contribute to membrane potential in a number of cell types including hypothalamic and hippocampal neurons, cardiac myocytes, vascular smooth muscle and neuroendocrine cells (Seino & Miki, 2003; Lam 2010; Hernandez-Sanchez et al., 2001; Engler & Yellon, 1996; Quayle et al., 1997; Proks et al., 2002). Importantly, K_{ATP} channels translate metabolic state to transmembrane potential and, in pancreatic beta cells, are central to glucose-stimulated insulin secretion (see e.g. Seino & Miki, 2003). Since electrical status is generally correlated to biological output in excitable tissues, JB253 provides a suitable tool for investigating K_{ATP} channel function under a range of normal and pathological states.

The prevailing view of sulfonylurea action is one of SUR1 binding, K_{ATP} channel closure and alterations to beta cell membrane potential (see e.g. Miki et al., 1999). However, recent studies have also invoked a K_{ATP}-independent signaling pathway whereby sulfonylurea may alter insulin release via Epac2A interactions (see e.g. Zhang et al., 2009; Herbst et al., 2011). Using radioactive displacement assays in combination with FRET experiments, JB253 was found to interact with both SUR1 and Epac2A. While SUR1 affinity for JB253 was much lower than glimepiride, we were unable to properly assess the active *cis*-state due to rapid thermal back-relaxation. As such, a role for illumination in strengthening any interaction cannot be excluded *e.g*. by altering binding conformation due to isomerization. Nonetheless, JB253 and glimepiride possess similar *EC₅₀* values for intracellular Ca²⁺ rises and, when applied at the same concentration, both compounds stimulated almost identical levels of insulin secretion. Thus, JB253 possesses a similar activity profile to glimepiride, most likely due to signaling via pathways generally acknowledged to underlie sulfonylurea action.

In addition to photopharmacology, optogenetic and artificial light-sensitive K⁺ channels are equally applicable to the remote control of electrically-responsive cells, including beta cells (Reinbothe et al., 2014). However, therapeutic potential in humans is limited by the requirement for genetic manipulation, high activation irradiances and the hyperpolarizing effects of recombinantly-expressed Kir6.2. Alternatively, an implantable synthetic optogenetic transcription device has recently been shown to improve blood-glucose homeostasis in a mouse model of T2DM *via* the expression and secretion of incretin (Ye at al., 2011). However, debate still exists as to whether incretin-based therapies are associated with increased risk of pancreatitis and pancreatic adenocarcinoma (Nauck & Friedrich, 2013). Nonetheless, similar concepts have recently been extended to designer fusion molecules and may in the future be adopted for insulin release (Rossger et al., 2013). By contrast, due to its favorable profile as an exogenously-applied sulfonylurea which is sensitive to light, JB253 has advantages both as a research tool and as an anti-diabetic agent.

In the context of photodynamic therapy, light penetration in human tissues has been studied in detail and is now well-understood (Grossweiner et al., 2005). Although, the current activation wavelength of JB253 (400-500 nm) limits deep tissue penetration, *e.g*. through the skin, variants of the photoswitchable sulfonylureas according to the invention can be used which can be switched at longer wavelengths. In addition, stimulated by the brisk development of optogenetics (Deisseroth 2011), devices that can deliver light to target tissues with minimal invasiveness and high spatial precision have emerged (Baretto et al, 2011; Kim et al., 2013).

Therefore, the photoswitchable sulfonylureas according to the invention, such as compound JB253, or related photoswitchable molecules which regulate K_{ATP} channels, will have an impact on human medicine and research. A long-standing challenge in endocrinology has been the inability to properly recreate the dynamics that underlie pulsatile hormone release, a prerequisite for proper downstream organ function (Seino et al., 2011). Furthermore, disparate biological systems can use similar or identical molecular components. For example, K_{ATP} channels are also expressed in the heart and brain, including in neuronal populations tasked with the central regulation of glucose homeostasis and counterregulatory responses (Lam 2010; Pocai et al., 2005). Photopharmacology has the ability to target drug activity to the primary site of dysfunction with high spatial and temporal resolution (Velema et al., 2014). JB253 holds particular promise in this regard. It is non-cytotoxic and can be used to repeatedly modulate rodent and human beta cell activity, the basis for recreating the oscillatory activity known to orchestrate hormone pulse, Its light-dependency means that JB253 activity is spatially-restricted by illumination, potentially reducing extra-pancreatic effects. Finally, the ability to 'turn on' or 'turn off JB253 action allows insulin secretion to be tailored to peak demand. Therefore, the photoswitchable sulfonylureas according to the invention, such as compound JB253, open up new avenues for the treatment of T2DM.

In summary, we have designed and synthesized light-sensitive sulfonylureas, in particular compound JB253, which have a broad spectrum of application due to conferment of photoswitching on K_{ATP} activity.

The following examples and drawings illustrate the present invention without, however, limiting the same thereto.

### BRIEF DESCRIPTION OF THE DRAWINGS

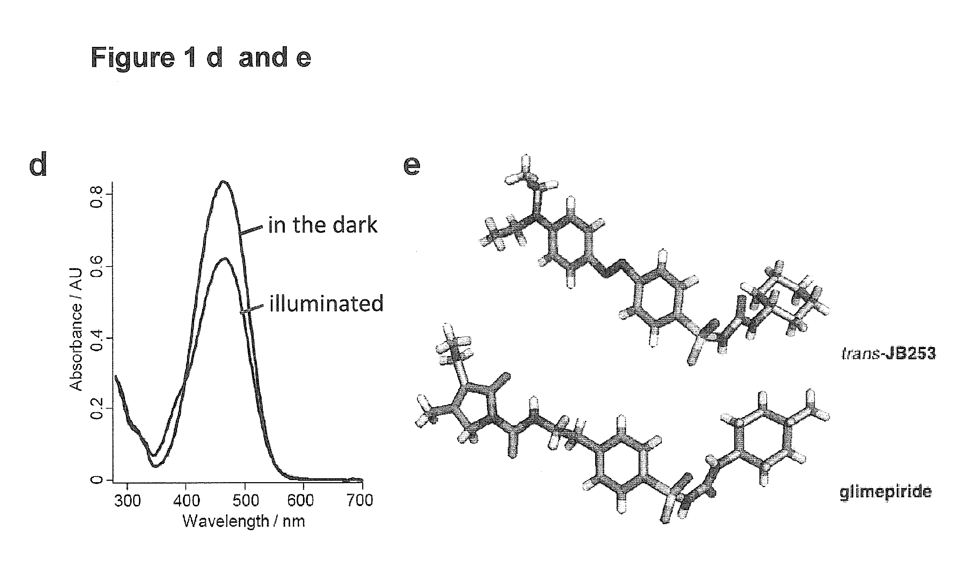

**Figure 1****:** *Photopharmacology of K_{ATP} channels: design, synthesis and characteristics of JB253.*
   a) The logic of a photoswitchable sulfonylurea: upon photoisomerization to the *cis*-state, JB253 becomes more active, closing the K_{ATP} channel. Thermal relaxation makes the compound less active or leads to dissociation, restoring the open form of the channel. Closure of K_{ATP} channels leads to depolarization, promoting calcium influx and ultimately insulin release.
   b) Chemical structure of tolbutamide and glimepiride, which served as templates for JB253.
   c) Synthesis, structure and switching characteristics of JB253. Sulfanilamide undergoes diazotization and is trapped with *N,N*-diethylaniline to yield an azobenzene-sulfonamide, which is converted to JB253 by cyclohexyl isocyanate. *trans*-JB253 can be reversibly switched to *cis*-JB253 with blue light and relaxes thermally.
   d) UV/Vis spectra of JB253 in the dark (black) and during constant illumination with 460 nm (blue).
   e) Crystal structure of *trans*-JB253 (CCDC: 1014606) and glimepiride (CSD: TOHBUN01) showing the structural similarity of both sulfonylureas.

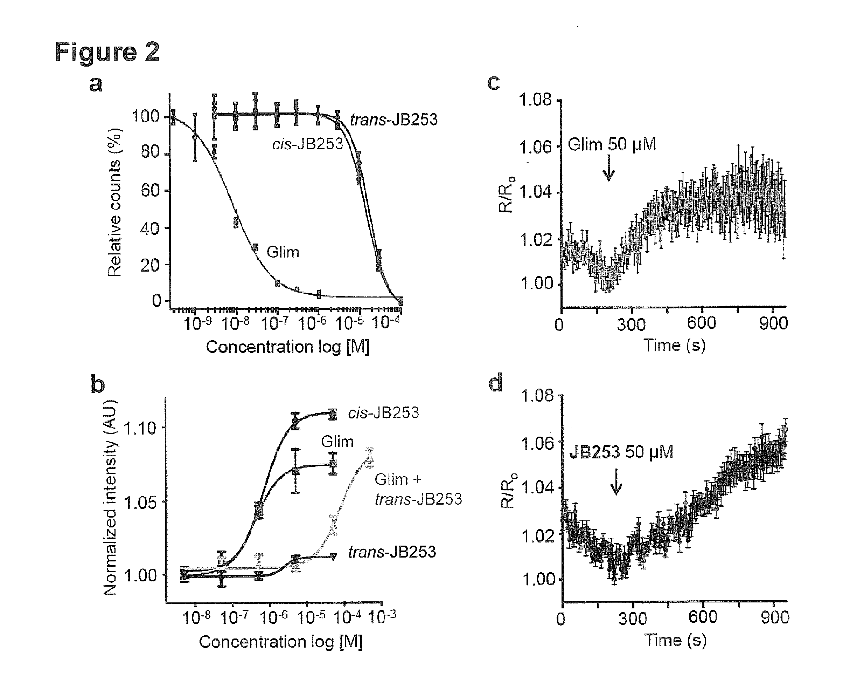

**Figure 2****:** *JB253 binding and concentration-response studies.*
   a) Binding affinity of glimepiride (Glim) (red, *IC₅₀* = 8.3 nM), *trans*-JB253 (black, *IC₅₀* = 17.6 µM) and *cis*-JB253 (blue, *IC₅₀* = 14.8 µM) were indirectly determined in SUR1-expressing HEK293t cells using displacement of [3H]-glibenclamide (datapoints fitted using the Hill equation) (*n* = 3 independent repeats). Note that, due to the potential for thermal dark-relaxation during the harvesting and washing steps, effects of photoswitching on JB253 binding affinity could not be excluded. Values represent the mean ± SD.
   b) *cis*-JB253 and glimepiride (Glim) possess similar concentration-response curves for the stimulation of [Ca²⁺]ᵢ in mouse islets, while *trans*-JB253 is largely ineffective. The concentration-response for glimepiride is right-shifted in the presence of a saturating concentration (100 µM) of *trans*-JB253 (datapoints fitted using non-linear regression) (*n* = 3-5 recordings).
   c) HEK293t cells expressing a full length Epac2-camps probe respond to glimepiride with decreases in *Förester resonance energy transfer* (FRET) (represented here as an increase in R/R₀) (*n* = 28 cells from 4 recordings).
   d) As for c) but *cis*-JB253. Values represent the mean ± SEM for b)-d).

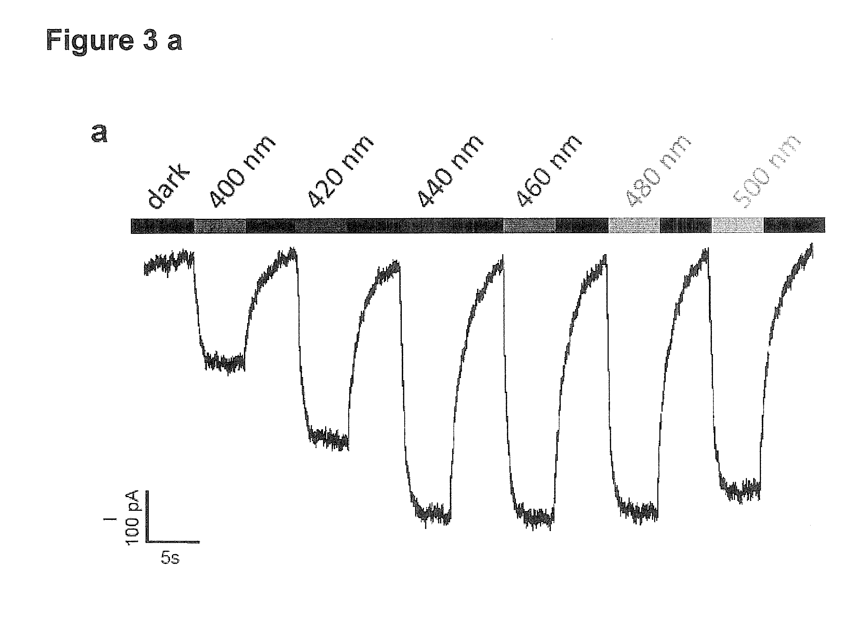

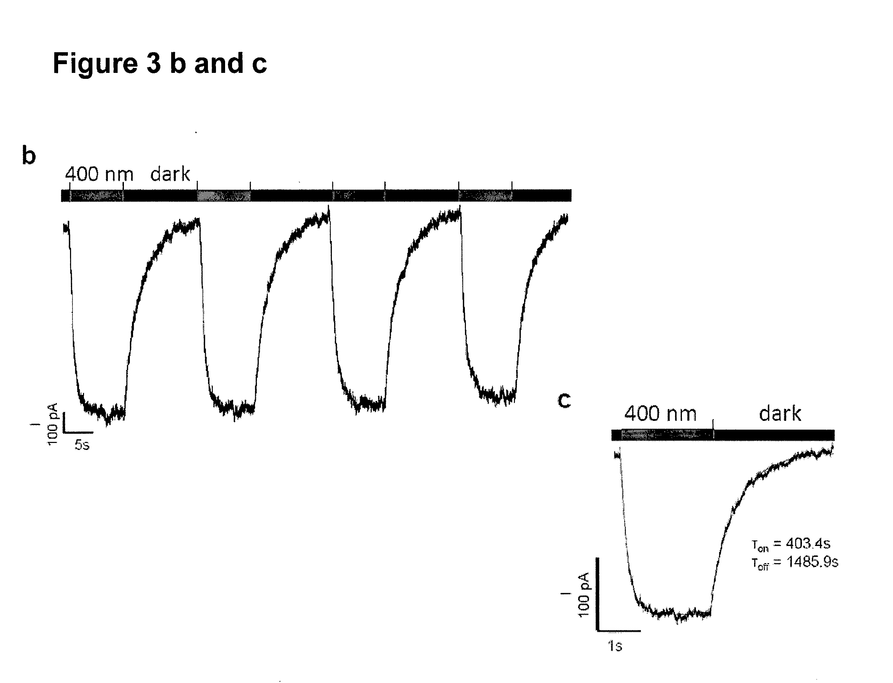

**Figure 3****:** *JB253 confers photoswitching on K_{ATP} channels:*
   a) Photocurrents recorded from HEK293t cells transfected with plasmids encoding Kir6.2 and SUR1 using the whole cell patch clamp configuration (holding potential -60 mV). **JB253-**treated cells respond to wavelengths between 400-500 nm with a reduction in the magnitude of the K⁺ inward rectifier current.
   b) JB253 allows reversible and repeated closure of K_{ATP} channels in response to light-dark cycles using λ = 400 nm to induce *cis*-isomerization (purple) and relaxation in the dark (black).
   c) Kinetics of light-triggered block and thermal restoration of K_{ATP} channel currents mediated by JB253.
   In all cases, traces represent *n* = 4 cells.

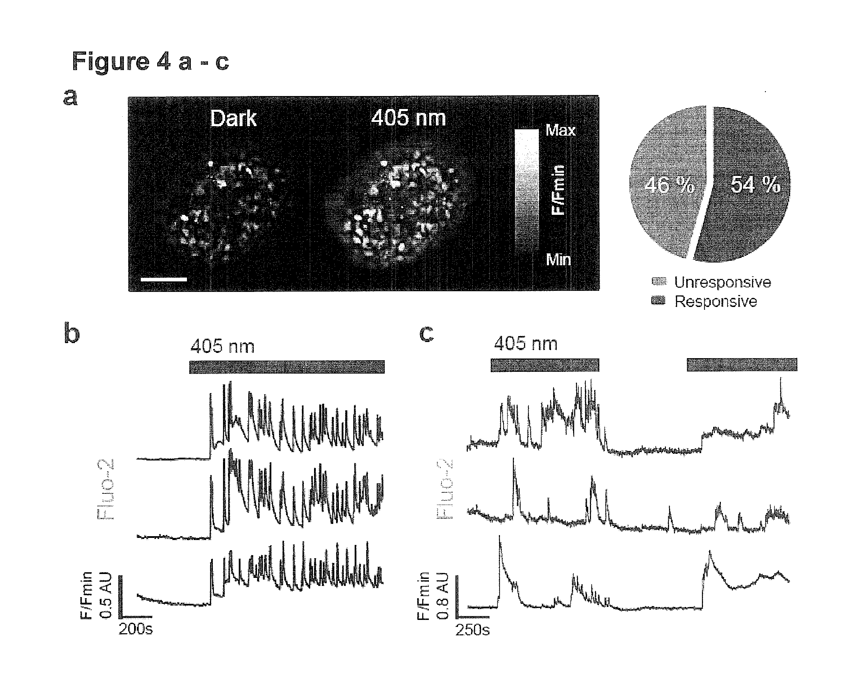

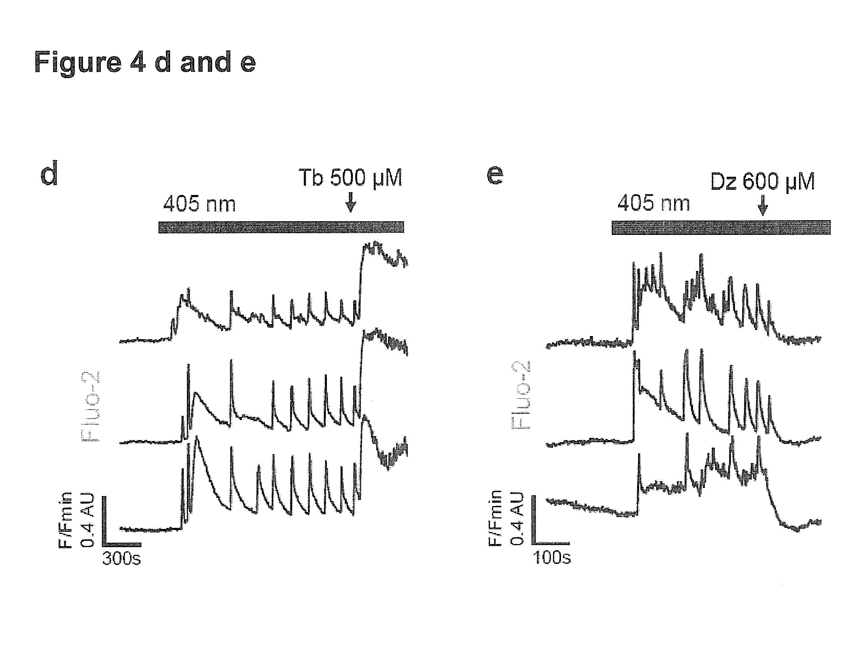

**Figure 4****:** *JB253 allows optical manipulation of pancreatic beta cell activity.*
   a) Beta cells residing within JB253-treated islets display large increases in cytosolic Ca²⁺ following exposure to 405 nm (purple) (scale bar, 110 µm) (linear LUT).
   b) Illumination with 405 nm induces large and synchronous rises in Ca²⁺ as indicated by Fluo-2 (representative traces from *n* = 10 recordings).
   c) JB253 can be used to impose complex dynamics including Ca²⁺ oscillations (representative traces from *n* = 5 recordings).
   d) High dose tolbutamide (Tb) augments the effects of JB253 on K_{ATP} channel blockade (representative traces from *n* = 6 recordings).
   e) Diazoxide (Dz) inhibits JB253 effects by opening the K_{ATP} channel ion pore (representative traces from *n* = 4 recordings).

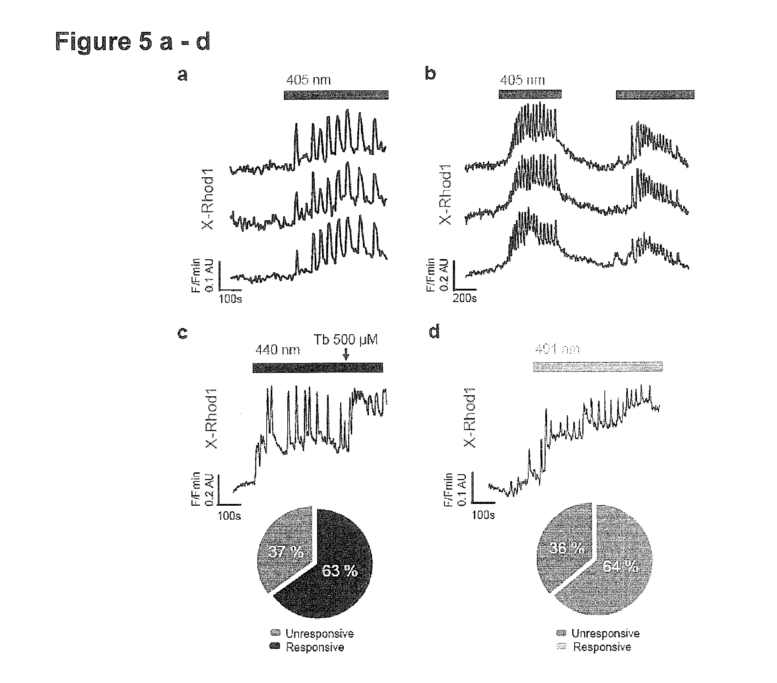

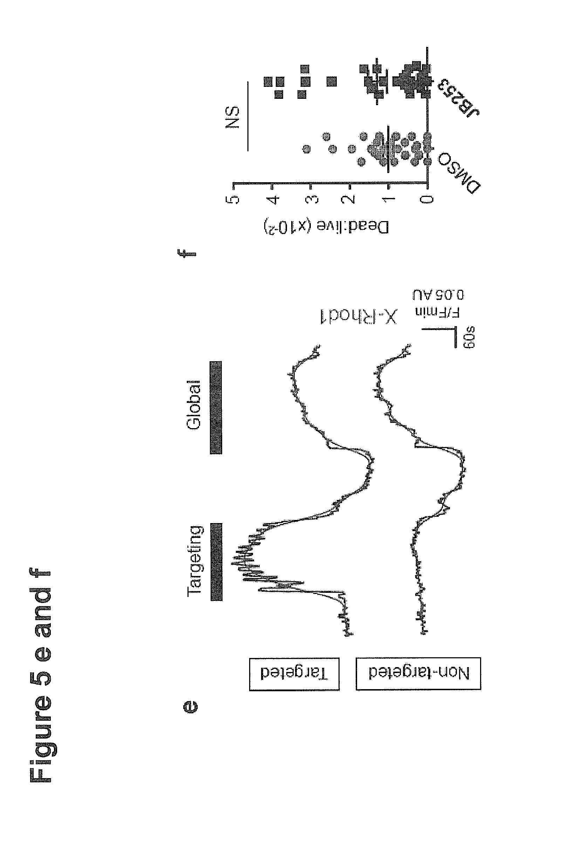

**Figure 5****:** *Specific photoswitching of beta cell function in the violet-blue spectrum.*
   a) JB253-treated cells loaded with the red-shifted Ca²⁺ indicator X-Rhod1 (λ excitation = 561 nm) similarly respond to 405 nm with Ca²⁺ rises (representative traces from *n* = 3 recordings).
   b) As for a) but imposition of oscillations (representative traces from *n* = 3 recordings).
   c) JB253-treated beta cells display large increases in cytosolic Ca²⁺ following exposure to 440 nm (representative traces from *n* = 6 recordings).
   d) As for c), but following illumination with 491 nm (Tb, tolbutamide; positive control) (representative traces from *n* = 5 recordings).
   e) A single islet can be photoswitched using a targeting laser while leaving its neighbor quiescent (∼ 200 (µm center-center) (representative traces from *n* = 3 recordings). A global laser pulse evokes activity in both islets (grey, raw; red, smoothed).
   f) Incubation of islets with JB253 does not alter cell viability as assessed by calcein AM and propidium iodide incorporation (NS, non-significant *versus* DMSO-alone, Student's t-test) (*n* = 28 islets from four animals). Values represent mean ± SEM.

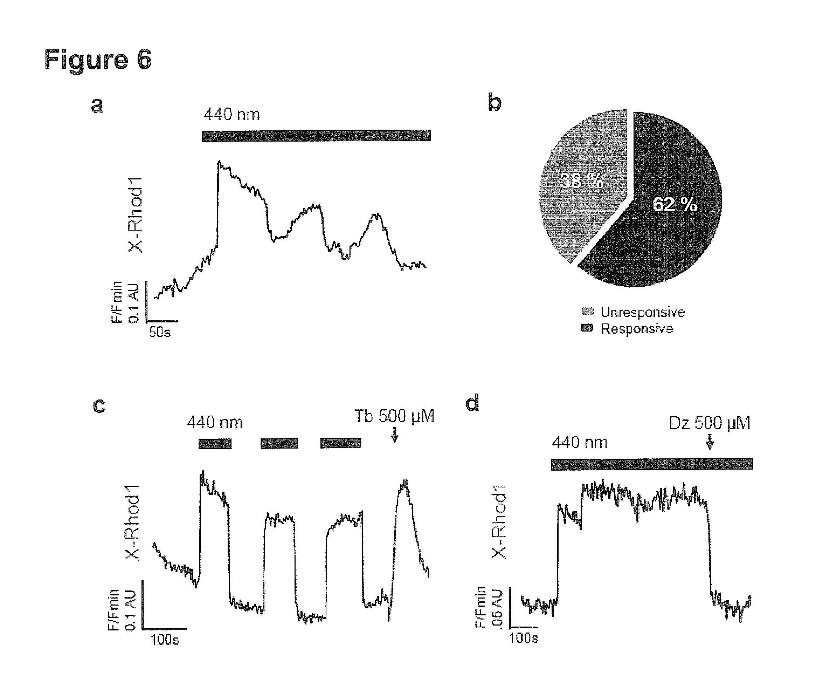

**Figure 6****:** *Manipulation of human islet activity using JB253.*
   a) **JB253**-treated beta cells residing within intact human islets respond to 440 nm with rapid rises in cytosolic Ca²⁺ levels (representative traces from *n* = 8 recordings).
   b) Almost 62% of X-Rhod1-loaded cells respond to JB253 with Ca²⁺ rises.
   c) As for a) but imposition of oscillations to demonstrate reversibility of JB253 effects in human tissue (Tb, tolbutamide; positive control) (*n* = representative traces from 4 recordings);
   d) Reversal of JB253 action using diazoxide to open the K_{ATP} channel ion pore (Dz, diazoxide; negative control) (*n* = representative traces from 5 recordings). In all cases, islets were derived from three donors.

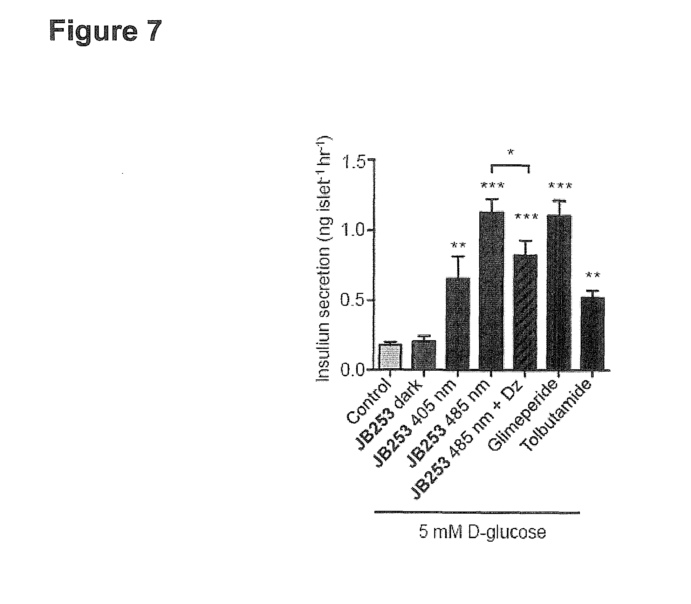

**Figure 7****:** *JB253 yields optical control of insulin secretion.*
   Application of JB253 under dark conditions is unable to influence insulin release *versus* control (5 mM glucose-alone) during static incubation of isolated murine islets. By contrast, illumination with 405 nm and 485 nm significantly increases insulin secretion, and this is similar in magnitude to that achieved using glimepiride and tolbutamide (*n* = 9 mice) (*P<0.05 *versus* JB253 485 nm; **P<0.01 and ***P<0.001 *versus* G5; one-way ANOVA). Values represent mean ± SEM.
**Figure 8**
   a) ¹H NMR of JB253.
   b) ¹³C NMR of JB253.
   c) 2D NMR of JB253 (HSQC)
   d) 2D NMR of JB253 (HMBC).

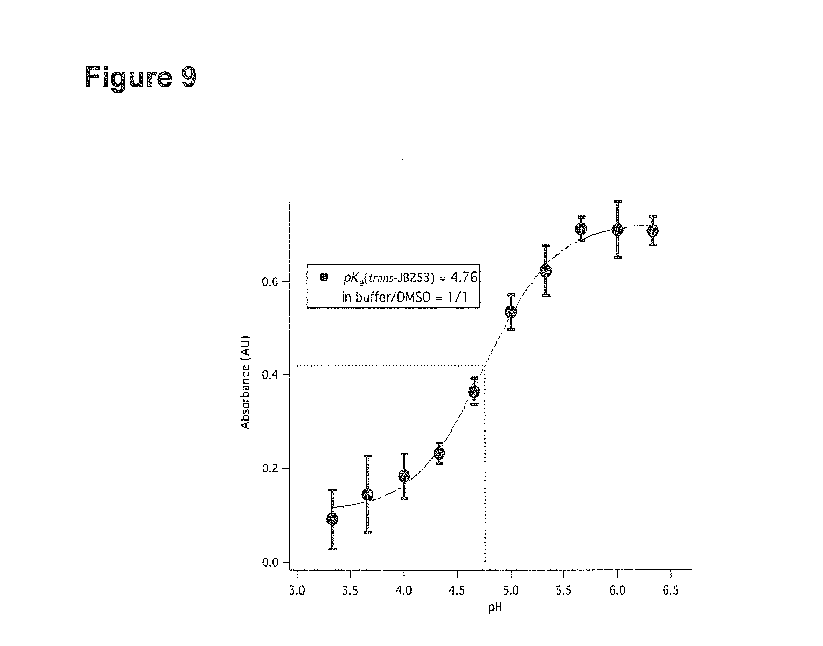

**Figure 9****:** *pKₐ measurement of JB253.*
   Ionization constant of *trans*-JB253 was determined with an absorbance-based platereader assay according to Martinez & Dardonville (2013) in a DMSO/buffer mixture (1/1). All datapoints were acquired in triplicate (*n* = 3 assays) at the following pH-values: 3.33, 3.66, 4.00, 4.33, 4.66, 5.00, 5.33, 5.66, 6.00 and 6.33. Ionic strength was held constant (*I* = 0.1 mM) by the addition of KCl to each buffer. Data was background subtracted and the spectral differences plotted against the pH. Sigmoidal fitting (IgorPro v6.22a) obtained a *pKₐ* = 4.76. Values represent mean ± SD.

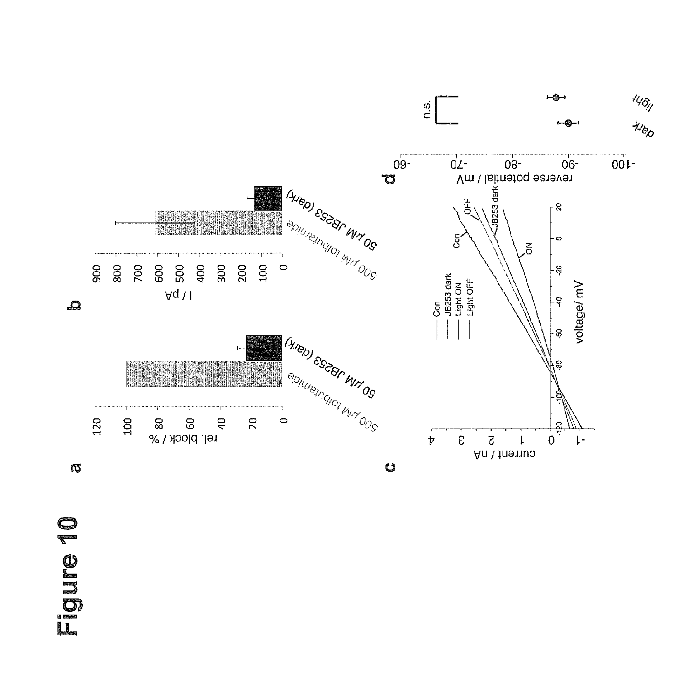

**Figure 10****:** *K_{ATP} channel block characteristics of JB253 in the dark*.
   a) To account for cell-cell variation in current densities, magnitude block with JB253 (dark) was calculated as a percentage *versus* that achieved with 500 µM tolbutamide in the same experiment (% ± SEM; *n* = 3 recordings).
   b) Bar graph displaying the amplitude of the inward current (ΔI [pA]) at -60 mV elicited by application of either tolbutamide or JB253 in the dark (current ± SEM, *n* = 3 recordings).
   c) Representative current-voltage (IV) relationships showing a minor decrease in membrane conductance upon application of JB253 in the dark (before drug = black; after drug = orange). This inhibition is reversibly enhanced by exposure to blue light (blue = ON; pink = OFF).
   d) Mean reversal potential (Eᵣₑᵥ) before and after illumination of JB253 measured in recordings as depicted in c). (NS, non-significant, *trans*-JB253 *versus cis*-JB253; Student's paired t-test) (mV ± SEM, *n* = 5 recordings).

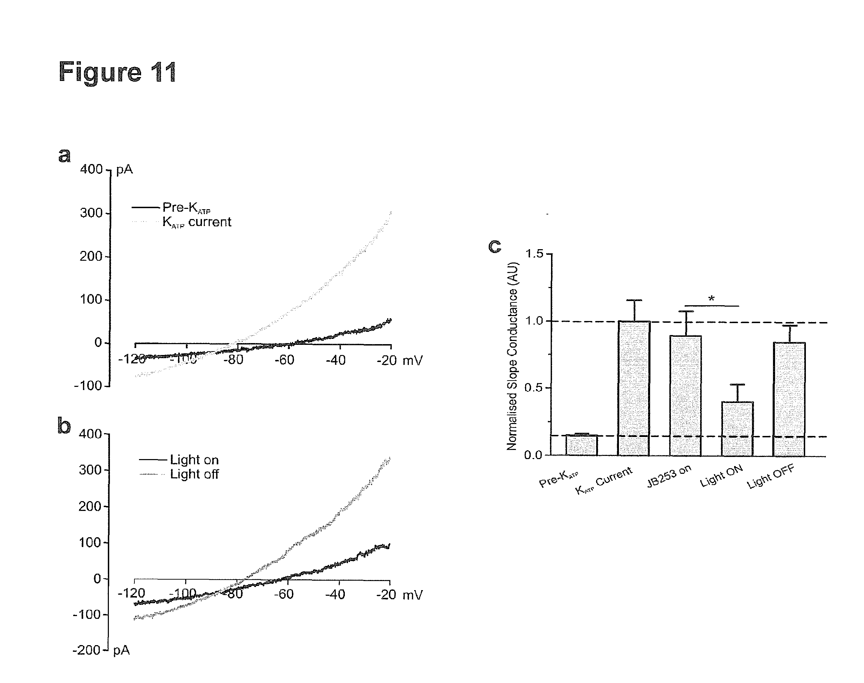

**Figure 11****:** JB253 reversibly blocks K_{ATP} currents in MIN6 beta cells.
   a) Representative current-voltage (IV) relationships recorded straight after establishing the whole-cell configuration (Pre-K_{ATP}) and after development of the K_{ATP} current due to washout of ATP from the cell (Peak K_{ATP} current).
   b) IVs from the same cell as a) in the presence of either *trans*-JB253 (light off) or *cis*-JB253 (light on).
   c) Bar graph displaying mean data from experiments as shown in a) and b). Slope conductance was normalized to peak K_{ATP} current. Note that at this concentration (10 mM) JB253 only blocks the K_{ATP} current during illumination and this is readily reversed when the light source is shut off (*P<0.05 *versus* JB253 dark; one-way ANOVA) (*n* = 4 recordings).
**Figure 12****:** *Extended JB253 action spectrum.*
   JB253 is unable to photoswitch K_{ATP} currents in HEK293t cells transfected with Kir6.2 and SUR1 at wavelengths > 560 nm (holding potential -60 mV) (trace representative of *n* = 3 recordings).
**Figure 13****:** *Extinction coefficient measurement of JB253.*
   Absorbance at two wavelengths (405 and 485 nm) was measured of a dilution series of JB253 (concentrations in µM: 0.01, 0.10, 1.00, 10.0, 25.0 50.0) in low K⁺ buffer (containing in mM: 3 KCl, 118 NaCl, 25 NaHCO₃, 2 CaCl₂, 1 MgCl₂, 10 HEPES, NaOH to pH 7.4) (*n* = 4). Data was background subtracted and the absorbance values plotted against the concentration to obtain the extinction coefficients *via* linear fitting. Values represent mean ± SD.

### EXAMPLES

### Example 1 Methods

### 1.1 Chemical synthesis

### (E)-4-((4-(diethylamino)phenyl)diazenyl)benzenesulfonamide

Sulfanilamide (2.00 g, 11.61 mmol, 1.0 eq.) was dissolved in 2.4 M HCl and cooled to 0 °C. Under vigorous stirring, a solution of NaNO₂ (0.96 g, 13.91 mmol, 1.2 eq.) in 6 mL water was added drop wise until the solution turned pale yellow. The formed diazonium salt was stirred under ice-cooling for an additional 10 minutes before it was transferred into a solution of *N,N-*diethylaniline (1.73 g, 11.61 mmol, 1.84 mL, 1.0 eq.) in a 1/1 mixture of 1 M NaOAc/MeOH. The solution turned to dark red and was allowed to warm to r.t. under stirring. The crude product was extracted with EtOAc (3x), and the combined organic layers were washed with brine and dried over MgSO₄. Flash column chromatography (25% EtOAc/*i*-hexanes) yielded 1.45 g (4.37 mmol) of the desired product as a red powder in 38% yield.

¹H NMR (400 MHz, DMSO-d₆): δ [ppm] = 7.95 (d, *J* = 8.6 Hz, 2H), 7.88 (d, *J* = 8.6 Hz, 2H), 7.81 (d, *J =* 9.2 Hz, 2H), 7.45 (s, 2H), 6.82 (d, *J =* 9.3 Hz, 2H), 3.47 (q, *J* = 7.0 Hz, 4H), 1.15 (t, *J* = 7.0 Hz, 6H). ¹³C NMR (101 MHz, DMSO-d₆): δ [ppm] = 154.2, 150.8, 143.8, 142.2, 126.9, 125.8, 121.9, 111.1, 44.2, 12.5. HRMS (ESI): calc. for C₁₆H₂₁N₄O₂S⁺ (M+H)⁺: 333.1380, found: 333.1377. Rₜ (LCMS; MeCN/H₂O/formic acid = 10/90/0.1 → 90/10/0.1 over 7 min) = 4.364 min. UV/Vis (LCMS): λₘₐₓ = 460 nm.

### (E)-N-(cyclohexylcarbamoyl)-4-((4-(diethylamino)phenyl)diazenyl)benzenesulfonamide (JB253)

A mixture of (*E*)-4-((4-(diethylamino)phenyl)diazenyl)benzenesulfonamide (332 mg, 1.0 mmol, 1.0 eq.) and Cs₂CO₃ (1.30 g, 4.0 mmol, 4.0 eq.) in acetone (20 mL) was refluxed for 1 h before addition of cyclohexyl isocyanate (125 mg, 1.0 mmol, 119 µL, 1.0 eq.) diluted in acetone (20 mL). The reaction mixture was refluxed for an additional 3 h, before cooling to ∼40 °C. The crude solid was filtered and washed with small amounts of acetone before it was carefully dissolved in MeOH to yield 450 mg (0.98 mmol) of JB253 product in 98% yield.

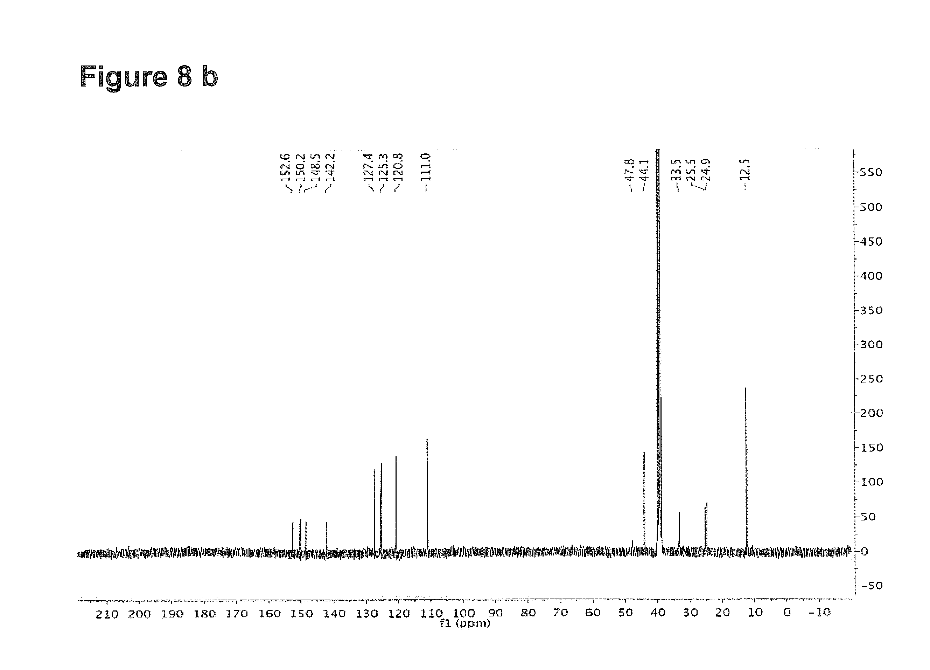

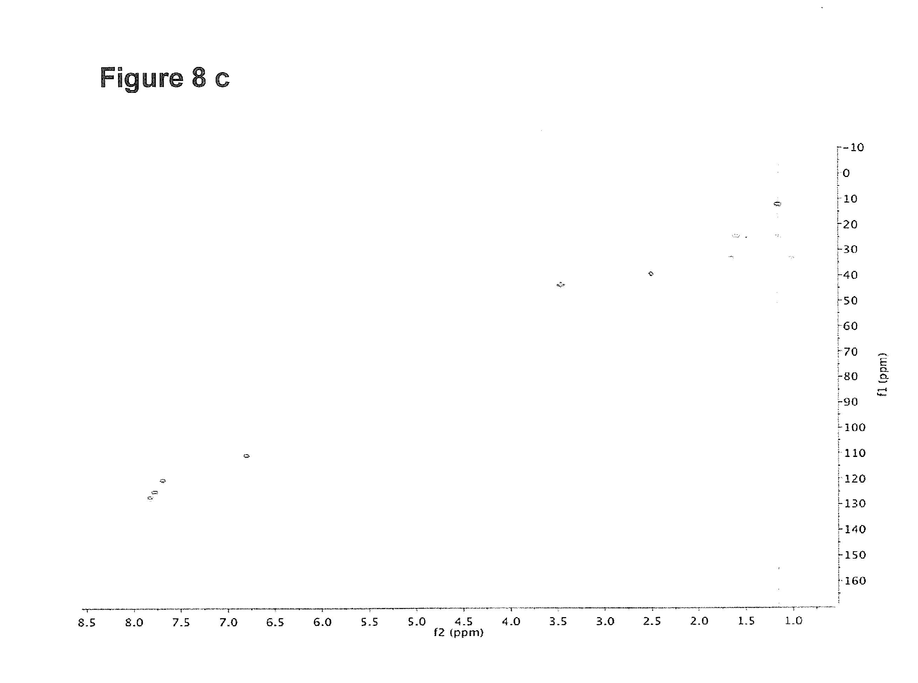

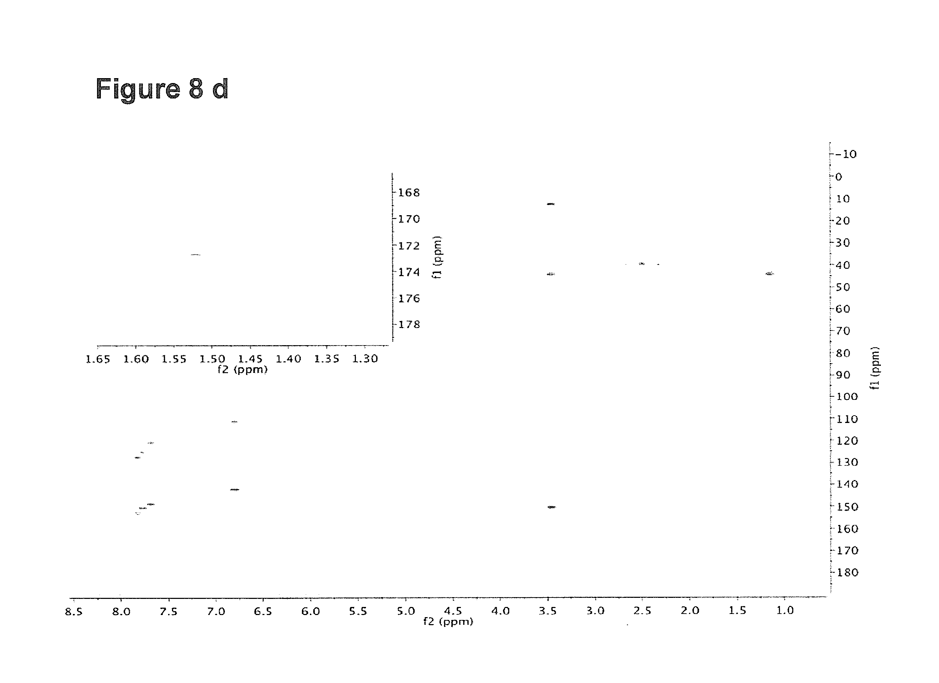

¹H NMR (400 MHz, DMSO-d₆): δ [ppm] = 7.83 (d, *J* = 8.5 Hz, 2H), 7.78 (d, *J* = 9.1 Hz, 2H), 7.69 (d, *J* = 8.5 Hz, 2H), 6.80 (d, *J =* 9.3 Hz, 2H), 5.62 (br s, 2H), 3.46 (q, *J* = 7.0 Hz, 4H), 3.20 (br s, 1H), 1.86-1.38 (m, 5H), 1.33-0.92 (m, 11H). ¹³C NMR (101 MHz, DMSO-d₆): δ [ppm] = 172.7 (HMBC, see Figure 8d), 152.6, 150.2, 148.5, 142.2, 127.4, 125.3, 120.8, 111.0, 47.8, 44.1, 33.5, 25.5, 24.9, 12.5. HRMS (ESI): calc. for C₂₃H₃₂N₅O₃S⁺ (M+H)⁺: 458.2220, found: 458.2219. Rₜ (LCMS; MeCN/H₂O/formic acid = 10/90/0.1 → 90/10/0.1 over 7 min) = 5.285 min. UV/Vis (100 µM in DMSO): λₘₐₓ = 472 nm; (LCMS): λₘₐₓ = 468 nm. ε₄₀₅ nm = 18,501 mol⁻¹ cm⁻¹; ε₄₈₅ nm = 38,670 mol⁻¹ cm⁻¹. IR (ATR): wavenumber/cm⁻¹ = 3331, 2928, 2851, 1652, 1626, 1602, 1576, 1537, 1514, 1390, 1349, 1174, 1130, 1086, 1042, 843, 820, 676. m.p. = 190 °C.

### 1.2 General Chemistry

Flash column chromatography was carried out on silica gel 60 (0.040-0.063 mm) purchased from Merck. RP flash column chromatography was carried out on Waters C18 silica gel (0.055-0105 mm, 125 Å). Reactions and chromatography fractions were monitored by thin layer chromatography (TLC) on Merck silica gel 60 F254 glass plates. The spots were visualized either under UV light at 254 nm or with appropriate staining method (iodine, *p-*anisaldehyde, KMnO₄) followed by heating.

NMR spectra were recorded in deuterated solvents on Varian Mercury 200, Bruker AXR 300, Varian VXR 400 S, Bruker AMX 600 and Bruker Avance III HD 400 (equipped with a CryoProbe™) instruments and calibrated to residual solvent peaks (¹H/¹³C in ppm): DMSO-d₆ (2.50/39.52). Multiplicities are abbreviated as follows: s = singlet, d = doublet, t = triplet, q = quartet, br = broad, m = multiplet. Spectra are reported based on appearance, not on theoretical multiplicities derived from structural information.

A Varian MAT CH7A mass spectrometer was used to obtain low- and high-resolution electron impact (EI) mass spectra. Low- and high-resolution electrospray (ESI) mass spectra were obtained on a Varian MAT 711 MS instrument operating in either positive or negative ionization modes.

Solvents for column chromatography and reactions were purchased in HPLC grade or distilled over an appropriate drying reagent prior to use. If necessary, solvents were degassed either by freeze-pump-thaw or by bubbling N₂ through the vigorously stirred solution for several minutes. Unless otherwise stated, all other reagents were used without further purification from commercial sources.

UV/Vis spectra were recorded on a Varian Cary 50 Bio UV-Visible Spectrophotometer using Helma Suprasil precision cuvettes (10 mm light path).

LC-MS was performed on an Agilent 1260 Infinity HPLC System, MS-Agilent 1100 Series, Type: 1946D, Model: SL, equipped with a Agilent Zorbax Eclipse Plus C18 (100 x 4.6 mm, particle size 3.5 micron) RP column.

Infrared spectra were recorded on a Perkin Elmer Spectrum BX-59343 instrument as neat materials. For detection a Smiths Detection DuraSam-plIR II Diamond ATR sensor was used. The measured wave numbers are reported in cm⁻¹.

Melting points were measured on an EZ-Melt apparatus (Stanford Research Systems) and are uncorrected.

Extinction coefficients were measured on a BMG Labtech Omega Series FLUOstar microplate reader with clear flat-bottom white 96 wellplates by full spectra acquirement in low K⁺ external bath buffer (containing in mM: 3 KCl, 118 NaCl, 25 NaHCO₃, 2 CaCl₂, 1 MgCl₂, 10 HEPES; NaOH to pH 7.4). All JB253-containing solutions (dilution series, n = 4: 10 nM; 100 nM; 1 µM; 10 µM; 25 µM; 50 µM) were background substracted and fitted with a linear slope. Volumes were 100 µL each, which resulted in a path length of *l* = 2.94 mm. *pKₐ* Measurements and data processing were performed using the same instrument and protocol according to Martinez & Dardonville (2013). Full absorbance spectra (280-800 nm) were acquired and background subtracted before spectral differences were calculated. The total change of maximal positive and maximal negative difference was calculated and plotted against pH. Sigmoidal fit of the obtained plot gave access to the *pKₐ*.

### 1.3 Crystallography

X-Ray data collection was performed on a Bruker D8Venture at 173 K using MoKα-radiation (λ = 0.71073 Å). The APEX2 (v2012.4-3, Bruker AXS Inc.) software and embedded programs were applied for the integration, scaling and multi-scan absorption correction of the data. The structures were solved by direct methods with SIR97 (Altomare et al., 1999) and refined by least-squares methods against F2 with SHELXL-97 (Sheldrick et al., 2008). All non-hydrogen atoms were refined anisotropically. The C-bound hydrogen atoms were placed in ideal geometry riding on their parent atoms, N-bound hydrogen atoms were refined freely. The crystallographic data for JB253 is available free of charge from The Cambridge Crystallographic Data Centre *via* www.ccdc.cam.ac.uk/data request/cif (accession ref. CCDC 1014606).

### 1.4 Electrophysiology

HEK293t cells (obtained from Leibniz-Institut DSMZ: #305) were incubated in Dulbecco's Modified Eagle Medium (DMEM) + 10% FBS and used for electrophysiological recordings 24-48 hrs following Lipofectamine transfection with plasmids encoding Kir6.2 (Genbank D50581), rat SUR1 (Genbank L40624) and GFP. Whole cell patch clamp experiments were performed using a standard electrophysiology setup equipped with a HEKA Patch Clamp EPC10 USB amplifier and PatchMaster software (HEKA Electronik). Micropipettes were generated from "Science Products GB200-F-8P with filament" pipettes using a vertical puller. Resistance varied between 5-7 MΩ. Bath solution contained in mM: 3 KCl, 118 NaCl, 25 NaHCO₃, 2 CaCl₂, 1 MgCl₂, 10 HEPES (NaOH to pH 7.4). Pipette solution contained in mM: 90 K-gluconate, 10 NaCl, 10 KCl, 1 MgCl₂, 10 EGTA, 60 HEPES (KOH to pH 7.3), and the holding potential was -60 mV. Illumination during electrophysiology and UV/Vis experiments was provided by a TILL Photonics Polychrome 5000 monochromator. JB253 was applied at 50 µM, as this concentration was found to be maximal for stimulating Ca²⁺ rises.

MIN6 cells (obtained from Dr Jun-ichi Miyazaki, Osaka University, see Miyazaki et al., 1990)) were cultured in DMEM supplemented with 15% FBS, 1% glutamine, 2% HEPES buffer, 0.0005% β-mercaptoethanol and 1% penicillin/streptomycin. Cells were seeded onto glass coverslips 24 hrs before whole cell patch clamp experiments using micropipettes generated from thin-walled borosilicate capillaries (3-6 MΩ). Bath solution contained in mM: 3 KCl, 118 NaCl, 25 HEPES, 3 MgCl₂ and 2 CaCl₂. Pipette solution contained in mM: 150 KCl, 3 MgCl₂, 5 EGTA, 10 HEPES, 0.3 K₂ATP (pH 7.2), and the holding potential was -60 mV. Illumination was provided using a X-Cite 120 mercury arc lamp (Lumen Dynamics) with a bandpass filter (470 ± 20 nm). Voltage ramps from -20 mV to -120 mV (500 ms duration) were applied every 5 s to produce current-voltage relationships in the presence and absence of JB253. All cells lines were regularly mycoplasma tested.

### 1.5 Mouse islet isolation

Male and female CD1 and C57BL6 mice (8-20 weeks) were maintained in a specific pathogen-free facility under a 12h light-dark cycle with ad libitum access to water and food. Animals were euthanized using a schedule-1 method and pancreatic islets isolated by collagenase digestion. All procedures were regulated by the Home Office according to the Animals (Scientific Procedures) Act 1986 of the United Kingdom (PPL 70/7349), and study approval granted by the *Animal Welfare and Ethical Review Body (AWERB) of* Imperial College. No randomization was used for animal experimentation, since mice were only used as tissue donors.

### 1.6 Human islet isolation

Human islets were isolated from deceased heart-beating donors (*n* = 3) at transplantation facilities in Pisa and Edmonton with the relevant national and local ethical permissions, including consent from next of kin where required, and cultured in RPMI supplemented with 5.5 mM D-glucose, 10% foetal calf serum (FCS), 100 U penicillin, 100 µg streptomycin and 0.25/µl fungizone (37 °C, 5% CO₂). All studies involving human tissue were approved by the National Research Ethics Committee (NRES) London (Fulham), REC # 07/H0711/114.

### 1.7 Calcium imaging

Islets were loaded for 30-45 min in fluo2-AM (10 µM) or for 2-5 min with X-Rhod 1 (5 µM) diluted with a mixture of DMSO (0.01%, wt/vol) and pluronic acid (0.001%, wt/vol; all Invitrogen) in a bicarbonate buffer containing in mM: 120 NaCl, 4.8 KCl, 1.25 NaH₂PO₄, 24 NaHCO₃, 2.5 CaCl₂, 1.2 MgCl₂ and 5 D-glucose. fMCI was performed using a Zeiss Axiovert M200 fitted with a Nipkow spinning-disk head (Yokogawa CSU-10) and a 10x/0.3NA objective adjusted for chromatic aberration (EC Plan-Neofluar, Zeiss). Pulsed excitation (frequency = 0.5 Hz; exposure = 263 ms) was delivered at 491 nm and emitted signals recorded at 500-550 nm with a back-illuminated 16-bit EM-CCD camera (ImageEM 9100-13; Hamamatsu). During recording, islets were maintained at 35-36 °C in the presence of 50 µM JB253 using a custom-manufactured perfusion and heating system (Digital Pixel). Drugs were introduced through the perfusion system at the indicated time points and concentrations. Violet light was delivered by a 405 ± 5 nm laser coupled to the side port of the microscope and configured to fill the back of the objective with light using an Optospot (Cairn Research). Blue light was delivered using 440 ± 5 nm and 491 ± 5 nm diode lasers controlled by a laser merge module (Spectral Applied Research) to allow simultaneous exposure and acquisition. For single islet targeting, a 473 ± 5 nm laser was coupled to a custom-manufactured dichroic array (Cairn Research), allowing user-directed steering of a collimated laser spot across the field of view. Signals were normalized using F/Fmin where F is fluorescence at a given timepoint and Fmin is minimum fluorescence.

### 1.8 Cytotoxicity assay

Islets were incubated with either DMSO or JB253 for 1 hr before staining with 3 µM of calcein-AM (live) and 2.5 µM of propidium iodide (dead). Absorbance/emission was detected at 491/525 nm and 561/620 nm for calcein and PI, respectively. The area of dead:live cells was calculated as a unitary ratio and the observer blinded to treatment identity.

### 1.9 EPAC2 imaging

For EPAC2 imaging, HEK293t were transfected with the full length construct for Epac2-camps containing the cAMP and sulfonylurea binding domains (obtained from Prof. Jin Zhang, Johns Hopkins University, see Herbst et al., 2011) before imaging (Hodson et al., 2014) using a HEPES-bicarbonate buffer containing in mM: 120 NaCl, 4.8 KCl, 24 NaHCO₃, 0.5 Na₂HPO₄, 5 HEPES, 2.5 CaCl₂, 1.2 MgCl₂ and 5 D-glucose. Excitation was delivered at 440 nm and emitted signals captured using cerulean (530 nm) and citrine (470 nm) filters. FRET was calculated as the ratio of Cerulean (CFP):Venus (YFP) fluorescence. Signals were normalized using R/R₀ where R is the ratio at a given timepoint and R₀ is the minimum ratio.

### 1.10 Measurements of insulin secretion from isolated islets

Insulin secretion was measured from 6 islets per well, incubated at 37 °C for 30 min in 0.5 ml of Krebs-HEPES-bicarbonate (KHB) solution (containing in mM: 130 NaCl, 3.6 KCl, 1.5 CaCl₂, 0.5 MgSO₄, 0.5 NaH₂PO₄, 2 NaHCO₃, 10 HEPES, and 0.1 % (wt/vol) BSA, pH 7.4) containing the indicated glucose concentration and tolbutamide (100 µM), glimeperide (50 µM), diazoxide (250 µM) and JB253 (50 µM). Illumination (λ = 405 ± 20 nm and 485 ± 6 nm) was performed using a Fluostar Optima microplate reader (BMG Labtech) set to deliver 30 s of light every 2 min to the designated wells. Insulin concentrations were determined in duplicate using specific radioimmunoassay (EMD Millipore).

### 1.11 [3H]-Glibenclamide radioassay

SUR1-expressing HEK293t cells were harvested and washed twice in assaying buffer containing in mM: 119 NaCl, 4.7 KCl, mM CaCl₂, 1.2 KH₂PO₄, 1.2 MgSO₄, 5 NaHCO₃, and 20 HEPES, pH 7.4. In a 96-well plate, ∼200,000 cells/well were incubated for 50 min with [3H]-glibenclamide (PerkinElmer) and different concentrations of glimepiride (Sigma-Aldrich) or JB253. Incubation was terminated by rapid filtration through Whatman GF/C filters by means of a Brandel MWXR-96 TI harvester and filters were washed 3 times with ice-cold assay buffer. Radioactivity was counted 6 h after cell and filter lysis in 200 µL Rotiszint EcoPlus (Roth) using a Packard microbeta scintillation counter (PerkinElmer).

### 1.12 Statistical analysis

Data distribution was determined using the D'Agostino omnibus test. Non-multifactorial pairwise comparisons were made using either the Student's t-test. Interactions between multiple treatments were assessed using one-way ANOVA followed by pairwise comparisons using Bonferroni's post-hoc test. Effect sizes in islets/cells are usually sufficiently large that multiple animals/independent replication is a more important determinant of power in studies requiring statistical comparison. Non-linear regression was used to calculate the EC₅₀ of normalized and log-transformed concentration-response curves. For [3H]-glibenclamide displacement assays, data points were fitted to the Hill equation before calculation of the halfmax value. In all cases, analysis was performed using Graphpad Prism (Graphpad Software) and IgorPro, and experimental numbers reported as independent biological replicates. No animals or data were excluded from the analysis and results were considered significant at P<0.05.

### Example 2 Results

### 2.1 Design and synthesis of JB253

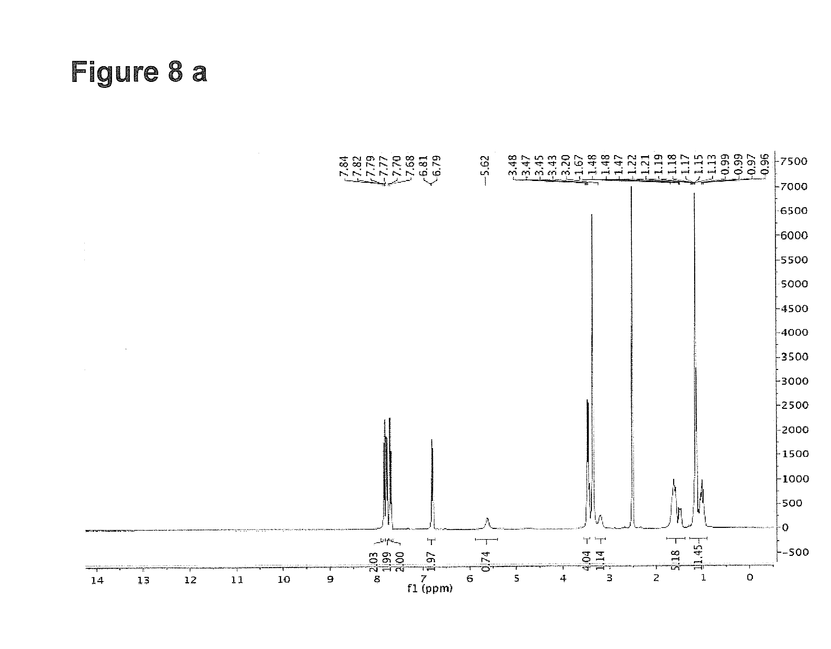

Distinct substitution patterns are found within different classes of arylsulfonylurea drugs: while there may be a variety of moieties on the aryl-ring, ranging from a simple methyl group in tolbutamide to more complex structures like a linked pyrrolidinone in glimepiride (Figure 1b), the terminal nitrogen in sulfonylureas is usually substituted with an aliphatic group. We reasoned that, to generate a photoswitchable analogue, the aromatic core of the sulfonylurea drugs could be extended to an azobenzene. Furthermore, we aimed for a cyclohexyl substituent on the urea moiety that mimics the corresponding substituent on glimepiride. Using a simple three-step procedure commencing with sulfanilamide, *N,N*-diethylaniline and cyclohexyl isocyanate, JB253 could be synthesized rapidly and inexpensively in large quantities *via* the sulfonamide-azobenzene (*E*)-4-((4-(diethylamino)phenyl)-diazenyl)benzenesulfonamide (Figure 1c) (Figure 8a-d). Initial photochromic characteristics of JB253 were measured using a UV/Vis spectrophotometer equipped with a monochromator, affording a single broad band as expected for a push-pull-azobenzene-system (λₘₐₓ = 472 nm) (Figure 1d).

Azobenzenes are known to be photoconverted between their *cis-* and *trans*-state by excitation with different wavelengths of light, or alternatively by illumination and dark relaxation. Indeed, JB253 was readily converted to its *cis*-state by applying wavelengths ranging from λ = 400-500 nm (peak λ = 472 nm), while thermal relaxation to its *trans*-state occurred rapidly in the dark. X-ray diffractometry revealed a high degree of structural similarity between *trans*-JB253 and glimepiride crystals (Figure 1e) (see Table 1). Whereas glimepiride in solution rotates freely around its ethylene carbon chain to adopt various possible binding conformations, JB253 is rigid unless illuminated and so can only adopt two conformations depending on isomeric state (*i.e. trans-* or *cis-*).

**Table 1: Crystallographic data for JB253**

| | **JB253** |
|---|---|
| net formula | C₂₃H₃₁N₅O₃S |
| *M*ᵣ/g mol⁻¹ | 457.590 |
| crystal size/mm | 0.100 × 0.080 × 0.050 |
| *T*/*K* | 173(2) |
| radiation | 'Mo Kα |
| diffractometer | 'Bruker D8Venture' |
| crystal system | monoclinic |
| space group | *P2*₁/*n* |
| *a*/Å | 15.8069(6) |
| *b*/Å | 9.0578(3) |
| *c*/Å | 17.1444(7) |
| α/° | 90 |
| β/° | 95.8298(13) |
| γ/° | 90 |
| *V*/Å³ | 2441.97(16) |
| *Z* | 4 |
| calc. density/g cm⁻³ | 1.24466(8) |
| µ/mm⁻¹ | 0.166 |
| absorption correction | multi-scan |
| transmission factor range | 0.9045-0.9585 |
| refls. measured | 26927 |
| *R*ᵢₙₜ | 0.0416 |
| mean σ(*I*)/*I* | 0.0408 |
| θ range | 3.28-27.52 |
| observed refls. | 4046 |
| *x, y* (weighting scheme) | 0.0742, 2.2726 |
| hydrogen refinement | mixed |
| refls in refinement | 5578 |
| parameters | 299 |
| restraints | 0 |
| *R*(*F*_{obs}) | 0.0601 |
| *R*_{w}(*F*²) | 0.1664 |
| *S* | 1.049 |
| shift/errorₘₐₓ | 0.001 |
| max electron density/e Å⁻³ | 1.022 |
| min electron density/e Å⁻³ | -0.402 |

Attaching lipophilic azobenzene units normally renders molecules poorly soluble in water and aqueous buffers, an obvious drawback for their use in biological systems. JB253, however, demonstrates excellent water solubility (≥ 0.1 mM) when diluted from a 50 mM stock solution in DMSO, presumably due to its acidity (*pKₐ* (*trans*-JB253) = 4.76; see Figure 9). These features were a promising entry point for our subsequent studies using mammalian tissue.

### 2.2 JB253 binding studies

To determine the binding affinity of JB253 to SUR1 relative to a known sulfonylurea (*i.e.* glimepiride), [3H]-glibenclamide displacement assays were performed. JB253 bound SUR1 with a 1000-fold lower affinity compared to glimepiride, and this was unaffected by illumination (*IC₅₀* = 8.3 nM *versus* 17.6 µM *versus* 14.8 µM for glimepiride *versus trans-*JB253 *versus cis*-JB253, respectively) (Figure 2a). However, due to the potential for rapid thermal dark-relaxation during the wash cycles (see below), we were unable to exclude a role for *trans-* to *cis-* isomerization in strengthening JB253 binding affinity. Therefore, to compare the activity profiles of *trans*-JB253, *cis*-JB253 and glimepiride using a functionally-relevant readout, concentration-response experiments were conducted in mouse islets. The *EC₅₀* of *cis-*JB253 for cytosolic Ca²⁺ rises was found to be 675 nM, similar to that obtained for glimepiride in the same system (*EC₅₀* glimepiride = 399 nM) (Figure 2b). The concentration-response curve for glimepiride was right-shifted in the presence of a saturating concentration of *trans*-JB253, demonstrating the presence of competitive agonism even under dark conditions (Figure 2b).

Since most sulfonlyureas have been reported to bind and activate Exchange Protein directly Activated by cAMP 2A (Epac2A), an important mediator of insulin secretion (see e.g. Herbst et al., 2011), the presence of interactions with JB253 was assessed using a Foster resonance energy transfer (FRET)-based approach. To enable this, a full length Epac2-camps biosensor containing the sulfonylurea binding site was encoded in HEK293t cells (Herbst et al., 2011). Confirming the existence of sufonylurea-Epac2A interactions, application of either glimepiride (Figure 2c) or *cis*-JB253 (Figure 2d) decreased FRET to a similar extent (ΔR/R₀ = 0.052 *versus* 0.064 AU, glimepiride *versus* JB253, respectively; NS, non-significant, Student's t-test).

### 2.3 JB253 allows photoswitching of K_{ATP} channels

We sought first to investigate whether JB253 could yield optical control over K_{ATP} channel activity using a system free from confounding effects of glucose metabolism. To enable this, K_{ATP} channels were heterologously expressed in HEK293t cells by transfection with plasmids encoding the Kir6.2 and SUR1 subunits along with GFP. Tolbutamide and diazoxide-sensitive inward-rectifying K⁺ currents could be recorded in transfected cells, confirming the functional assembly of K_{ATP} channels. In the dark state, JB253 partly reduced K⁺ current amplitude within a few seconds. This was however a fraction of that observed during 500 µM tolbutamide application (Figure 10a and b). Subsequent illumination of JB253 with wavelengths between 400-500 nm further closed the channel (Figure 3a), with ∼45-72% block being achieved relative to that recorded using 500 µM tolbutamide (Table 2). The reversal potential was close to the expected equilibrium potential for K⁺, and this was unaffected by molecule orientation (-90.0 ± 1.8 *versus* -87.8 ± 1.6 mV, dark *versus* illuminated; non-significant) (Figure 10c and d). As such, JB253 possesses the advantageous property of becoming a high affinity K_{ATP} channel blocker upon illumination.

Using a wavelength of 400 nm, heterologously-expressed K_{ATP} channels could be repeatedly opened and closed in JB253-treated preparations without obvious desensitization (difference in ΔI [pA] between first and last switch = 14.9 ± 7.6 %) (Figure 3b and c) (Supplementary Tables 3 and 4). Upon exposure to 400 nm, rapid block was observed (τₒₙ = 0.4 s) (Fig. 3c). When the light source was shut off, thermal relaxation was fast, returning the K_{ATP} channel to baseline levels within a couple of seconds (τ_{off} = 1.5 s) (Figure 3c) (see Tables 2 and 3). While maximal photoblock of K_{ATP} channels was observed at 460 nm, significant effects were also obtained with violet light (*i.e.* 405 nm), which was more compatible with fluorescence imaging of pancreatic beta cell function (see below). Confirming that JB253 could photoswitch endogenous K_{ATP} channels, hyperpolarizing currents were reversibly blocked in MIN6 beta cells following illumination (Figure 11).

**Table 2: Wavelength-dependent kinetics and current change in JB253-treated cells.**

| On/off kinetics for various wavelengths are shown in ms ± SD (*n* = 3 recordings). Current change is expressed as percentage (% ± SD)-block *versus* that obtained with tolbutamide in the same experiment (*n* = 3 recordings). Current change (ΔI [pA]) for a single representative experiment is displayed. In all cases, cells were exposed to 500 µM tolbutamide before washout and application of 50 µM JB253. | | | | |
|---|---|---|---|---|
| λ [nm] | τₒₙ [ms] | τ_{off} [ms] | % block | ΔI [pA] |
| 400 | 1176±385 | 2758±745 | 44.0±29.6 | 216 |
| 420 | 1309±438 | 2209±482 | 69.4±51.1 | 492 |
| 440 | 1246±421 | 2295±397 | 70.0±44.3 | 440 |
| 460 | 1181±447 | 1940±322 | 72.8±48.1 | 481 |
| 480 | 1163±434 | 2183±422 | 72.4±49.0 | 487 |
| 500 | 1244±462 | 2002±354 | 70.2±49.5 | 482 |

**Table 3: Kinetics and current change during a repetitive illumination cycle.**

| On/off kinetics are shown as ms ± SEM and current change as pA ± SEM calculated following four dark/400 nm cycles. Values are from a single cell. | | |
|---|---|---|
| τₒₙ [ms] | τ_{off} [ms] | ΔI [pA] |
| 477±26 | 1472±75 | 504.8±6.9 |

**Table 4: Current change for first and last switch for all experiments.**

| The difference in current change (ΔI [pA]) between the first and last switch (400nm) is represented as a percentage (%). | | | | |
|---|---|---|---|---|
| Experiment # | ΔI [pA] first switch | ΔI [pA] last switch | Cycles | Difference (%) |
| 1 | 136.5 | 89.5 | 12 | 34.4 |
| 2 | 438.8 | 423.8 | 9 | 3.4 |
| 3 | 200.0 | 161.0 | 5 | 19.5 |
| 4 | 521 | 510 | 4 | 2.1 |

### 2.4 Functional interrogation of beta cells within mouse islets

Stimulus-secretion coupling in beta cells relies on the closure of K_{ATP} channels, Ca²⁺ influx through VDCC and release of insulin granules. We therefore attempted to manipulate beta cell activity by optically controlling K_{ATP} channels with JB253.

Using functional multicellular Ca²⁺ imaging (fMCI) to monitor cell activity directly *in situ* within intact islets (Hodson et al., 2013; Rutter & Hodson, 2013), increases in cytosolic free Ca²⁺, assumed largely to emanate from beta cells under the conditions used here (Quesada et al., 1999), could be evoked following global illumination using a 405 nm laser (Fig. 4a) (*n* = 10 recordings). Just over half (54 %) of the fluo-2-loaded population responded to illumination with synchronous Ca²⁺ rises (Figure 4a and b). Demonstrating the utility of JB253 for the fine control of beta cell function, discrete Ca²⁺ oscillations, thought to underlie generation of insulin pulses, could be imposed using repeat exposure to 405 nm (Figure 4c). As anticipated, high doses of tolbutamide and diazoxide were able to augment and suppress, respectively, the effects of **JB253** (Figure 4d and e) (*n* = 4-6 recordings).

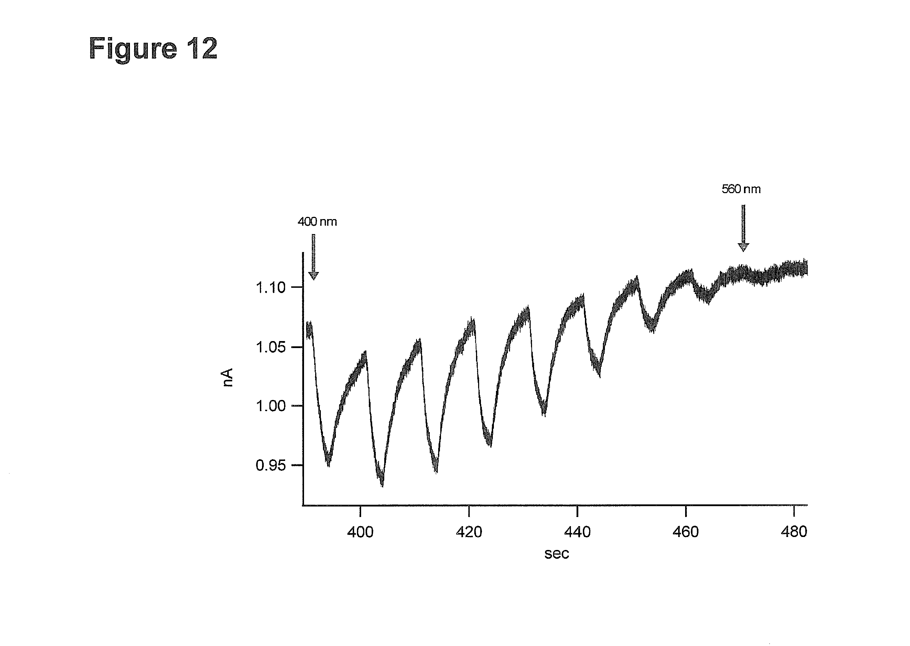

The wavelength required to excite Fluo-2 (λ = 491 nm), a commonly used Ca²⁺ indicator, could potentially lead to K_{ATP} channel closure in **JB253**-treated islets due to *cis*-isomer formation. We therefore decided to repeat the above experiments using the red (λ = 561 nm)-excited Ca²⁺ indicator X-Rhod-1. Identical results were obtained to studies with Fluo-2 (Figure 5a and b) (63% responsive X-Rhod-1-loaded cells) (*n* = 3 recordings), confirming that the photostationary state of JB253 during brief (263 ms) pulses of 491 nm light was alone insufficient to close K_{ATP} channels in the islet preparation. Likewise, global Ca²⁺ oscillations could be induced in JB253-treated islets by more prolonged illumination with 440 nm and 491 nm laser lines and, as expected from the electrophysiological recordings, both wavelengths appeared to activate a slightly larger cell population (Figure 5c and d). These observations were unlikely due to K_{ATP} channel closure at 561 nm, since JB253 was unable to photoswitch K⁺ currents at wavelengths > 560 nm (Figure 12).

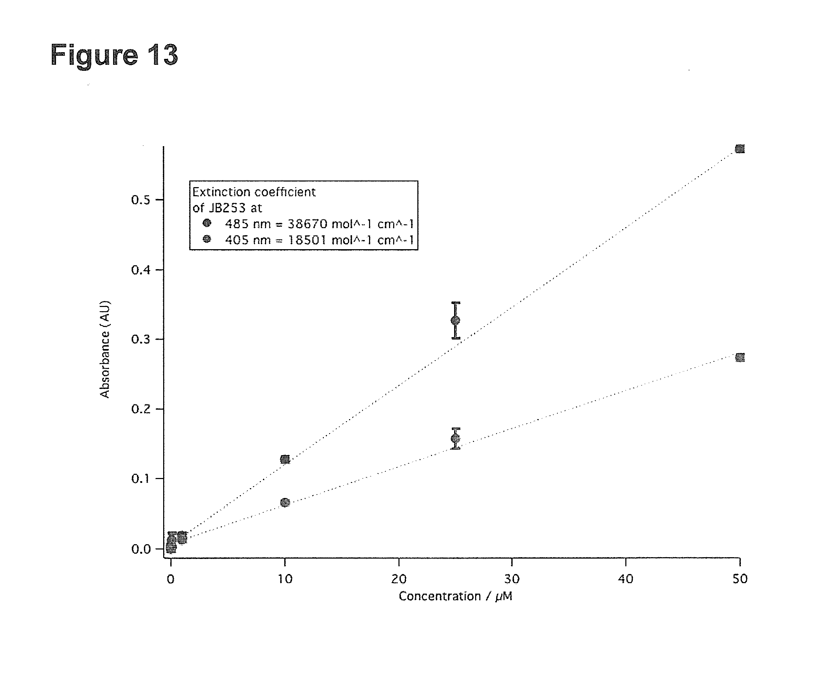

Demonstrating the spatial precision of JB253, a single islet from a doublet could be activated using a targeting laser without significantly stimulating its neighbor (∼ 200 µm from center to center) (Figure 5e), in part aided by the high molecule extinction coefficient (38,670 mol⁻¹ cm⁻¹ at 485 nm; see Figure 13). Lastly, JB253 did not appear to be cytotoxic to islets, as necrosis indices showed no significant differences in cell death *versus* DMSO-alone (Figure 5f).

### 2.5 Manipulation of human islet function using JB253

To underline the translational potential of JB253 for use in man, Ca²⁺-imaging experiments were repeated using isolated human islets of Langerhans. As observed for their mouse counterparts, beta cells within JB253-treated human islets responded to 440 nm and 491 nm with large intracellular Ca²⁺ rises, and oscillations could be coaxed simply by turning the laser on and off (Figure 6a-c). JB253 effects appeared to be due to K_{ATP} blockade, as they could be mimicked and reversed using tolbutamide and diazoxide, respectively (Figure 6c and d).

### 2.6 Optical stimulation of insulin release using JB253

To cement the link between photocontrol of K_{ATP} channels, [Ca²⁺]ᵢ and insulin secretion, islets (from n = 10 mice) were incubated in the presence of JB253 while exposing to either dark, 405 nm or 485 nm. Insulin release was similar in control experiments (5 mM glucose; shown to sensitize beta cells to sulfonylurea, Jonkers et al., 2001) and JB253-treated islets in the dark, suggesting that any K_{ATP} channel block and VDCC activity detected under these conditions was subthreshold for triggering Ca²⁺-activated exocytosis (Figure 7). By contrast, JB253-treated islets secreted almost 4-8-fold more insulin following illumination, and this could be partially reversed using diazoxide (Figure 7). When exposed to 485 nm light, JB253 was equipotent to glimepiride at stimulating insulin secretion (Figure. 7).

The features disclosed in the foregoing description, in the claims and/or in the accompanying drawings may, both separately and in any combination thereof, be material for realizing the invention in diverse forms thereof.

### REFERENCES

Altomare, A., et al. SIR97: a new tool for crystal structure determination and refinement. Journal of Applied Crystallography 32, 115-119 (1999).
Ashfield, R., F. M. Gribble, et al. (1999). "Identification of the high-affinity tolbutamide site on the SUR1 subunit of the K(ATP) channel." Diabetes 48(6): 1341-1347.
Barretto, R.P., et al. Time-lapse imaging of disease progression in deep brain areas using fluorescence microendoscopy. Nat. Med. 17, 223-228 (2011).
Broichhagen, J., Jurastow, I., Iwan, K., Kummer, W. & Trauner, D. Optical control of acetylcholinesterase with a tacrine switch. Angew. Chem. Int. Ed. Engl. 53, 7657-7660 (2014).
Currie, C.J., Poole, C.D. & Gale, E.A. The influence of glucose-lowering therapies on cancer risk in type 2 diabetes. Diabetologia 52, 1766-1777 (2009).
Deisseroth, K. Optogenetics. Nature methods 8, 26-29 (2011).
Engler, R.L. & Yellon, D.M. Sulfonylurea KATP blockade in type II diabetes and preconditioning in cardiovascular disease. Time for reconsideration. Circulation 94, 2297-2301 (1996).
Evans, J.M., Ogston, S.A., Emslie-Smith, A. & Morris, A.D. Risk of mortality and adverse cardiovascular outcomes in type 2 diabetes: a comparison of patients treated with sulfonylureas and metformin. Diabetologia 49, 930-936 (2006).
Fehrentz, T., Schonberger, M. & Trauner, D. Optochemical genetics. Angew. Chem. Int. Ed. Engl. 50, 12156-12182 (2011).
Fineman, M.S., et al. Effect on glycemic control of exenatide (synthetic exendin-4) additive to existing metformin and/or sulfonylurea treatment in patients with type 2 diabetes. Diabetes Care 26, 2370-2377 (2003).
Fortin, D.L., et al. Photochemical control of endogenous ion channels and cellular excitability. Nature methods 5, 331-338 (2008).
Gorostiza, P. & Isacoff, E.Y. Optical switches for remote and noninvasive control of cell signaling. Science 322, 395-399 (2008).
Grossweiner, L.I., Jones, L.R., Grossweiner, J.B. & Rogers, B.H.G. The science of phototherapy : an introduction, (Springer, Dordrecht; Norwell, MA, 2005).
Henquin, J.C. Regulation of insulin secretion: a matter of phase control and amplitude modulation. Diabetologia 52, 739-751 (2009).
Herbst, K.J., Coltharp, C., Amzel, L.M. & Zhang, J. Direct activation of Epac by sulfonylurea is isoform selective. Chem. Biol. 18, 243-251 (2011).
Hernandez-Sanchez, C., et al. Mice transgenically overexpressing sulfonylurea receptor 1 in forebrain resist seizure induction and excitotoxic neuron death. Proc. Natl. Acad. Sci. U. S. A. 98, 3549-3554 (2001).
Hodson, D.J., et al. Lipotoxicity disrupts incretin-regulated human beta cell connectivity. J. Clin. Invest. 123, 4182-4194 (2013).
Hodson, D.J., et al. ADCY5 couples glucose to insulin secretion in human islets. Diabetes (2014). DOI 10.2337/db13-1607.
Inagaki, N., T. Gonoi, et al. (1996). "A family of sulfonylurea receptors determines the pharmacological properties of ATP-sensitive K+ channels." Neuron 16(5): 1011-1017.
Jennings, A.M., Wilson, R.M. & Ward, J.D. Symptomatic hypoglycemia in NIDDM patients treated with oral hypoglycemic agents. Diabetes Care 12, 203-208 (1989).
Jonkers, F.C., Guiot, Y., Rahier, J. & Henquin, J.C. Tolbutamide stimulation of pancreatic beta-cells involves both cell recruitment and increase in the individual Ca(2+) response. Br. J. Pharmacol. 133, 575-585 (2001).
Kim, T.I., et al. Injectable, cellular-scale optoelectronics with applications for wireless optogenetics. Science 340, 211-216 (2013).
Lam, T.K. Neuronal regulation of homeostasis by nutrient sensing. Nat. Med. 16, 392-395 (2010).
Lemoine, D., et al. Optical control of an ion channel gate. Proc. Natl. Acad. Sci. U. S. A. 110, 20813-20818 (2013).
Martinez, C.H. & Dardonville, C. Rapid Determination of Ionization Constants (pK a) by UV Spectroscopy Using 96-Well Microtiter Plates. ACS medicinal chemistry letters 4, 142-145 (2013).
Miki, T., Nagashima, K. & Seino, S. The structure and function of the ATP-sensitive K+ channel in insulin-secreting pancreatic beta-cells. J. Mol. Endocrinol. 22, 113-123 (1999).
Miyazaki J, Araki K, Yamato E, Ikegami H, Asano T, Shibasaki Y, Oka Y, Yamamura K. Establishment of a pancreatic beta cell line that retains glucose-inducible insulin secretion: special reference to expression of glucose transporter isoforms. Endocrinology. 127(1):126-32 (1990).
Mourot, A., et al. Rapid optical control of nociception with an ion-channel photoswitch. Nature methods 9, 396-402 (2012).
Nathan, D.M., et al. Management of hyperglycemia in type 2 diabetes: A consensus algorithm for the initiation and adjustment of therapy: a consensus statement from the American Diabetes Association and the European Association for the Study of Diabetes. Diabetes Care 29, 1963-1972 (2006).
Nauck, M. A. and N. Friedrich (2013). "Do GLP-1-based therapies increase cancer risk?" Diabetes Care 36 Suppl 2: S245-252.
Pocai, A., et al. Hypothalamic K(ATP) channels control hepatic glucose production. Nature 434, 1026-1031 (2005).
Prentki, M. & Nolan, C.J. Islet beta cell failure in type 2 diabetes. J. Clin. Invest. 116, 1802-1812 (2006).
Proks, P., Reimann, F., Green, N., Gribble, F. & Ashcroft, F. Sulfonylurea stimulation of insulin secretion. Diabetes 51 Suppl 3, S368-376 (2002).
Quayle, J.M., Nelson, M.T. & Standen, N.B. ATP-sensitive and inwardly rectifying potassium channels in smooth muscle. Physiol. Rev. 77, 1165-1232 (1997).
Quesada, I., Nadal, A. & Soria, B. Different effects of tolbutamide and diazoxide in alpha, beta-, and delta-cells within intact islets of Langerhans. Diabetes 48, 2390-2397 (1999).
Rorsman, P., Braun, M. & Zhang, Q. Regulation of calcium in pancreatic alpha- and beta-cells in health and disease. Cell calcium 51, 300-308 (2012).
Rossger, K., Charpin-EI-Hamri, G. & Fussenegger, M. A closed-loop synthetic gene circuit for the treatment of diet-induced obesity in mice. Nature communications 4, 2825 (2013).
Rutter, G.A. & Hodson, D.J. Minireview: intraislet regulation of insulin secretion in humans. Mol. Endocrinol. 27, 1984-1995 (2013).
Seino, S., Shibasaki, T. & Minami, K. Dynamics of insulin secretion and the clinical implications for obesity and diabetes. J. Clin. Invest. 121, 2118-2125 (2011).
Seino, S. & Miki, T. Physiological and pathophysiological roles of ATP-sensitive K+ channels. Progress in Biophysics and Molecular Biology 81, 133-176 (2003).
Sheldrick, G. A short history of SHELX. Acta Crystallographica Section A 64, 112-122 (2008).
Stein, M., et al. Azo-propofols: photochromic potentiators of GABA(A) receptors. Angew. Chem. Int. Ed. Engl. 51, 10500-10504 (2012).
Stitt, A.W. AGEs and diabetic retinopathy. Invest. Ophthalmol. Vis. Sci. 51, 4867-4874 (2010).
Tochitsky, I., et al. Optochemical control of genetically engineered neuronal nicotinic acetylcholine receptors. Nature chemistry 4, 105-111 (2012).
Velema, W.A., et al. Optical control of antibacterial activity. Nature chemistry 5, 924-928 (2013).
Velema, W.A., Szymanski, W. & Feringa, B.L. Photopharmacology: beyond proof of principle. J. Am. Chem. Soc. (2014).
Zhang, C.L., et al. The cAMP sensor Epac2 is a direct target of antidiabetic sulfonylurea drugs. Science 325, 607-610 (2009).

## Claims

1. A sulfonylurea analogous compound comprising a sulfonylurea moiety and a photoresponsive moiety,
preferably having the general formula I
**Ph - SU**
wherein
**Ph** is the photoresponsive moiety, and
**SU** is the sulfonylurea moiety.

2. The sulfonylurea analogous compound of claim 1, wherein the photoresponsive moiety undergoes *trans-cis* isomerization by excitation with light, or by illumination and dark relaxation,
and/or wherein the *cis* state or isomer is active / is in the binding conformation to close ATP-sensitive potassium (K⁺) (K_{ATP}) channels, and the *trans* state or isomer is inactive, or vice versa..

3. The sulfonylurea analogous compound of claim 1 or 2, wherein the photoresponsive moiety is an azobenzene or a derivative thereof, such as heterocyclic azobenzene structure(s), substituted azobenzene structure(s) (e.g. tetra-ortho), bridged azobenzenes (e.g. by an o,o'-ethylene chain),
and/or wherein the sulfonylurea moiety and the photoresponsive moiety are covalently attached to each other..

4. The sulfonylurea analogous compound of any of claims 1 to 3, wherein the sulfonylurea moiety is derived from glibenclamide and glimepiride,
and/or wherein the sulfonylurea moiety contains substitutent(s) (such as aliphatic chains and/or rings, which can contain heteroatoms) on the terminal sulfonylurea nitrogen.

5. The sulfonylurea analogous compound of any of claims 1 to 4, wherein the activation wavelength is in the ultraviolet, visible and infrared light ranges of the electromagnetic spectrum.

6. The sulfonylurea analogous compound of any of the preceding claims, having the general formula II 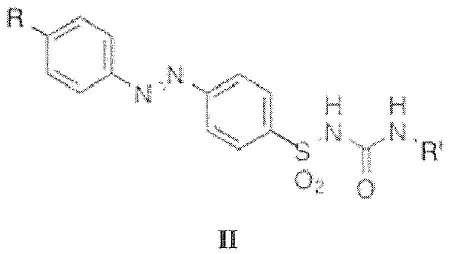 wherein
R is selected from hydrogen, alkyl, alkylamino, hydroxy or substituted hydroxy groups, morpholines and/or piperazines, and
R' is selected from cyclohexyl and substituted versions thereof, aliphatic linear chains and/or structures containing heteroatoms,
such as
(*E*)-*N*-(cyclohexylcarbamoyl)-4-((4-(diethylamino)phenyl)diazenyl)benzenesulfonamide (JB253)
and/or is selected from

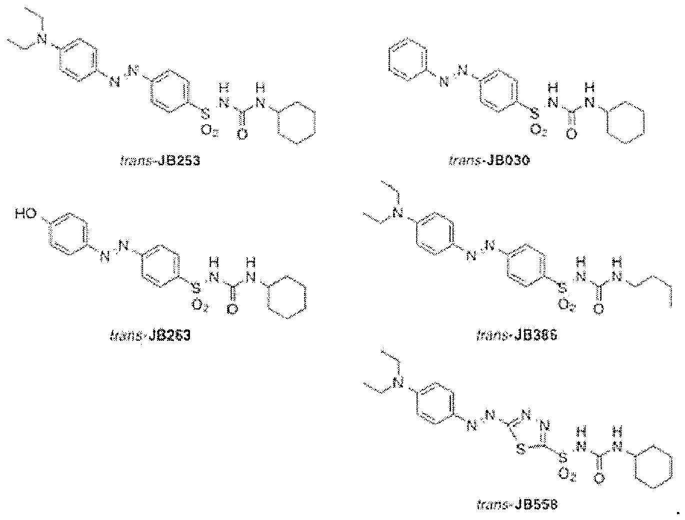

7. The sulfonylurea analogous compound of any of the preceding claims, comprising further moiety/moieties or component(s),
such as
- label (e.g. radioisotopes, MRI responsive groups, IR dyes, fluorophores)
- tag(s) (e.g. antibodies, peptides),
- anchoring group(s),
- other pharmacological units (e.g. biguanides, incretins)
and/or
- cell penetrating peptides (e.g. TAT).

8. A method for synthesizing/generating a sulfonylurea analogous compound of any of claims 1 to 7, comprising at least one of the following
the use of sulfanilamide and *N,N*-diethylaniline as the starting compounds,
the use of sulfanilamide and phenol as the starting compounds,
the use of 5-amino-1,3,4-thiadiazole-2-sulfonamide and *N,N*-diethylaniline as the starting compounds,
the use of nitroso compounds with anilines,
the use of hydrazines and coupling partners using Pd-chemistry,
via sulfonamide azobenzenes,
the further derivatization to a sulfonylurea by the use of aliphatic isocyanates (e.g. cyclohexyl, *n*-butyl, *trans*-4-methyl-cyclohexyl)
and/or
the use of any sulfonylchlorides thereof together with ureas.

9. A pharmaceutical composition, comprising
(i) at least one sulfonylurea analogous compound according to any of claims 1 to 7,
(ii) optionally, pharmaceutically acceptable excipient(s) and/or carrier(s),
(iii) optionally, further compound(s).

10. The sulfonylurea analogous compound of any of claims 1 to 7 or the pharmaceutical composition of claim 9 for use in medicine.

11. The sulfonylurea analogous compound or the pharmaceutical composition of claim 10 for use in the prevention and/or treatment of diabetes conditions or a disease state requiring modulation of ATP-sensitive potassium (K⁺) (K_{ATP}) channels,
wherein the diabetes conditions are preferably selected from
- type 1 diabetes,
- type 2 diabetes,
- maturity onset diabetes of the young (MODY),
- permanent neonatal diabetes mellitus (PNDM),
- latent onset autoimmune diabetes of adults (LADA),
- any disease state requiring restoration of normal glucose homeostasis,
- any disease state **characterized by** defective glucose tolerance, glucose counterregulation and/or insulin release.

12. The sulfonylurea analogous compound or the pharmaceutical composition of claim 10 or 11, either alone in combination with further compound(s), such as biguanide(s) or PPAR-gamma inhibitor(s).

13. The sulfonylurea analogous compound or the pharmaceutical composition of any of claims 10 to 12, comprising at least one of
- targeting of activity,
- spatially and temporally restricted activation by illumination,
- optical control of electrical activity,
- optical control over calcium fluxes,
- optical control over insulin release/secretion,
- optical control over glucagon release/secretion,
- optical control over somatostatin release/secretion,
- optical control over pancreatic peptide release/secretion,
- optical control over ghrelin release/secretion,
- repeated modulation of any cell/tissue expressing K_{ATP} channels,
- pulsatile or oscillatory insulin release/secretion,
- pulsatile or oscillatory activation of any cell/tissue expressing K_{ATP} channels,
- tailoring of insulin release/secretion to demand,
- optical control of exchange protein activated by cAMP (Epac).

14. The sulfonylurea analogous compound or the pharmaceutical composition of any of claims 10 to 13, comprising photodynamic therapy or photopharmacology.

15. Use of the sulfonylurea analogous compound of any of claims 1 to 10 for regulating ATP-sensitive potassium (K⁺) (K_{ATP}) channels and/or for assessing the functional role and/or activation of ATP-sensitive potassium (K⁺) (K_{ATP}) channels.
